(19) Europäisches Patentamt
European Patent Office
Office européen des brevets

(11) EP 3 027 764 B1

(12) **EUROPEAN PATENT SPECIFICATION**

(45) Date of publication and mention of the grant of the patent:
**12.10.2022 Bulletin 2022/41**

(21) Application number: **14832076.5**

(22) Date of filing: **31.07.2014**

(51) International Patent Classification (IPC):
***C12Q 1/06*** *(2006.01)* ***G01N 27/02*** *(2006.01)*
*G01N 27/327* *(2006.01)*

(52) Cooperative Patent Classification (CPC):
**G01N 27/028; G01N 27/026;** G01N 27/3278

(86) International application number:
**PCT/US2014/049228**

(87) International publication number:
**WO 2015/017695 (05.02.2015 Gazette 2015/05)**

(54) **PLANAR CONFORMAL CIRCUITS FOR DIAGNOSTICS**

KONFORME PLANARE SCHALTUNGEN ZUR DIAGNOSE

CIRCUITS CONFORMES PLANS POUR DIAGNOSTICS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.07.2013 US 201361860434 P**
**31.07.2013 US 201361860460 P**
**31.12.2013 US 201361922336 P**

(43) Date of publication of application:
**08.06.2016 Bulletin 2016/23**

(73) Proprietor: **The Board of Regents of The University of Texas System**
**Austin, TX 78701 (US)**

(72) Inventors:
- **PRASAD, Shalini**
**Richardson, TX 75080 (US)**
- **SELVAM, Anjan, Panneer**
**Richardson, TX 75080 (US)**

(74) Representative: **Potter Clarkson**
**The Belgrave Centre**
**Talbot Street**
**Nottingham NG1 5GG (GB)**

(56) References cited:
**WO-A1-2012/135683**
**US-A- 5 184 516**
**US-A1- 2004 043 502**
**US-A1- 2009 240 128**
**US-A1- 2009 242 425**
**US-A1- 2012 165 636**

- **JEROEN BROEDERS ET AL: "Miniaturised eight-channel impedance spectroscopy unit as sensor platform for biosensor applications", PHYSICA STATUS SOLIDI. A: APPLICATIONS AND MATERIALS SCIENCE, vol. 208, no. 6, 5 June 2011 (2011-06-05), pages 1357-1363, XP055348667, DE ISSN: 1862-6300, DOI: 10.1002/pssa.201001199**
- **JEROEN BROEDERS ET AL: "Mobile Application for Impedance-Based Biomimetic Sensor Readout", IEEE SENSORS JOURNAL, IEEE SERVICE CENTER, NEW YORK, NY, US, vol. 13, no. 7, 3 April 2013 (2013-04-03), pages 2659-2665, XP011513077, ISSN: 1530-437X, DOI: 10.1109/JSEN.2013.2256346**
- **ANJAN PANNEER SELVAM ET AL: "Design of a high sensitive non-faradaic impedimetric sensor", THE EFFECT OF APPLIED COMPRESSIVE LOADING ON TISSUE-ENGINEERED CARTILAGE CONSTRUCTS CULTURED WITH TGF-BETA3, IEEE, 28 August 2012 (2012-08-28), pages 3251-3254, XP032463633, ISSN: 1557-170X, DOI: 10.1109/EMBC.2012.6346658**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



**(Cont. next page)**

- JEROEN BROEDERS ET AL: "Embedded Unit for Point-of-Care Impedance Based Biosensor Readout", HIGH PERFORMANCE COMPUTING AND COMMUNICATION&2012 IEEE 9TH INTERNATIONAL CONFERENCE ON EMBEDDED SOFTWARE AND SYSTEMS (HPCC-ICESS), 2012 IEEE 14TH INTERNATIONAL CONFERENCE ON, IEEE, 25 June 2012 (2012-06-25), pages 1571-1577, XP032255245, DOI: 10.1109/HPCC.2012.229 ISBN: 978-1-4673-2164-8
- XIAOWEN LIU ET AL: "A fully integrated multi-channel impedance extraction circuit for biosensor arrays", IEEE INTERNATIONAL SYMPOSIUM ON CIRCUITS AND SYSTEMS. ISCAS 2010 - 30 MAY-2 JUNE 2010 - PARIS, FRANCE, IEEE, US, 30 May 2010 (2010-05-30), pages 3140-3143, XP031725193, ISBN: 978-1-4244-5308-5
- CAPITAN-VALLVEY ET AL.: 'Recent developments in handheld and portable optosensing: A review' ANALYTICA CHIMICA ACTA vol. 696, 2011, pages 27 - 46, XP055036093
- ADAM C SIEGEL ET AL: "Foldable Printed Circuit Boards on Paper Substrates", ADVANCED FUNCTIONAL MATERIALS, WILEY - V C H VERLAG GMBH & CO. KGAA, DE, vol. 20, no. 1, 8 January 2010 (2010-01-08), pages 28-35, XP001551577, ISSN: 1616-301X, DOI: 10.1002/ADFM.200901363 [retrieved on 2009-10-15]
- FERNANDO SERGIO RODRIGUES RIBEIRO TELES: "Biosensors and rapid diagnostic tests on the frontier between analytical and clinical chemistry for biomolecular diagnosis of dengue disease: A review", ANALYTICA CHIMICA ACTA, vol. 687, no. 1, 1 February 2011 (2011-02-01), pages 28-42, XP055286944, AMSTERDAM, NL ISSN: 0003-2670, DOI: 10.1016/j.aca.2010.12.011
- ANNI MÄÄTTÄNEN ET AL: "A low-cost paper-based inkjet-printed platform for electrochemical analyses", SENSORS AND ACTUATORS B: CHEMICAL, vol. 177, 1 February 2013 (2013-02-01), pages 153-162, XP055064216, ISSN: 0925-4005, DOI: 10.1016/j.snb.2012.10.113
- WIJITAR DUNGCHAI ET AL: "Electrochemical Detection for Paper-Based Microfluidics", ANALYTICAL CHEMISTRY, vol. 81, no. 14, 15 July 2009 (2009-07-15) , pages 5821-5826, XP055566856, US ISSN: 0003-2700, DOI: 10.1021/ac9007573

## Description

## BACKGROUND OF THE INVENTION

### 1. Field of the Invention

The present invention relates generally to the field of detection devices. More particularly, it concerns the use of paper microfluidics and handheld potentiostats to detect biomolecules and other target analytes.

### 2. Description of Related Art

The ability to design inexpensive and disposable diagnostics and analytical platforms that are also biodegradable is of great value to health care as well as the environment. It has been established that size based confinement of biomolecules is critical for achieving enhanced sensitivity in diagnostics. Typically, size based confinement is achieved through complex fabrication processes as used for complementary metal-oxide-semiconductor (CMOS) technologies, which increases the cost per unit and increases the effective cost of the technology. Low cost technologies use printed circuit boards which are difficult to dispose of and add costs to the environment due to poor biodegradability. Paper-based microfluidics have been developed that typically use screen printing technologies; however, issues remain with respect to achieving controlled fluid flow on top the surfaces.

Similarly, currently available market potentiostats are designed with the focus of applicability to a wide range of electrical/electrochemical techniques. This leads to bulky form factors and expensive components used in their construction. Moreover, they are designed to be used for electrochemical applications. Specific problems with such market potentiostats include the fact that they have large device form factors, making it difficult for use in point-of-care settings, have high noise at low current and low voltage settings, have expensive and repetitive software and firmware costs, have analog serial input/output interfaces, and have low robustness and non-universality in global application. On the other extreme, handheld portable potentiostats are very limited in customizability and applicability to a range of applications. Portable potentiostats are not noise efficient for biological applications and hence lack robustness. Specific problems with handheld potentiostats include high noise at low current and low voltage settings, low robustness for application to biosensing, and minimal operation choices for electrochemical applications.

BROEDERS, J. ET AL. "Miniturised eight-channel impedance spectroscopy unit as sensor platform for biosensor applications", Physica Status Solidi A, vol. 208, no. 6 June 2011, pages 1357-1363 describes a miniaturised, low cost impedance analyse to ease the use of impedance spectroscopy in biological setups.

BROEDERS, J. ET AL. "Mobile Application for Impedance-Based Biomimetic Sensor Readout", IEEE Sensors Journal, vol. 13, no. 7, April 2013, pages 2659 - 2665 describes a method for smartphone-based impedance spectroscopy, especially fine-tuned for biometric sensor readout.

SELVAM, A. P. ET AL. "Design of a high sensitive non-faradaic impedimetric sensor", 34th Annual International Conference of the IEEE EMBS, September 2012, pages 3251 - 3254 describes using a non-faradaic impedimetric sensor fabricated on a printed circuit board chip.

ADAM C SIEGEL ET AL: "Foldable Printed Circuit Boards on Paper Substrates", Adv. Funct. Mater. 2010, 20, 28-35 describes several low-cost methods for fabricating flexible electronic circuits on paper.

ANNI MAATTANEN ET AL: "A low-cost paper-based inkjet-printed platform for electrochemical analyses", Sensors and Actuators B 177 (2013j 153-162, describes an electrode platform printed on a recyclable low-cost paper substrate characterized using cyclic voltammetry.

WIJITAR DUNGCHAI ET AL: "Electrochemical Detection for Paper-Based Microfluidics" Anal. Chem. 2009, 81, 5821-5826, describes a demonstration of electrochemical detection for paper-based microfluidic devices.

Therefore, there remains a need for affordable, efficient, biodegradable diagnostic platforms.

## SUMMARY OF THE INVENTION

The claimed invention is a method of detecting or quantifying a target analyte in a sample using a handheld measuring device and a conformal analyte sensor circuit and is defined by the appended claims.

The claimed method uses conformal analyte sensor circuits comprising a porous nanotextured substrate and a conductive material situated on the top surface of the solid substrate in a circuit design, thereby creating a circuit comprising a working electrode and a reference electrode. The porosity of the nanotextured substrate is determined by the target analyte to be measured. In some embodiments, the porous nanotextured substrate has a porosity at or between $10 \times 10^5$ and $10 \times 10^{20}$ pores/cm$^2$. In some embodiments, the porous nanotextured substrate has a porosity at or between $10 \times 10^7$ and $10 \times 10^{16}$ pores/cm$^2$. In some embodiments, the porous nanotextured substrate is an insulating substrate. In some embodiments, the porous nanotextured substrate is paper or nitrocellulose.

In some embodiments, the porous nanotextured substrate includes hydrophobic coatings. In some embodiments, the hydrophobic coatings include parylene, polyamide, PEG, polycation solutions, and polydimethlysiloxane. In some embodiments, the porous nanotextured substrate includes surface coatings. In some embodiments, the surface coatings include pre-formulated sprays and aerosols that may induce hydrophobicity on specific regions of the sensor substrates. In some embodiments, the surface coatings include a mixture of ethanol, polydimethlysiloxane, ethyl sulfate, chlorotrimethylsilane, siloxanes and silicones as well as pre-formulated block co-polymer mixtures. In some embodiments, the porous nanotextured substrate includes track etched membranes. In some embodiments, the track etched membranes include nucleopore and cyclopore form factors. In some embodiments, the porous nanotextured substrate includes acid etched membranes. In some embodiments, the acid etched membranes include silicon and alumina scaffolds. In some embodiments, the porous nanotextured substrate includes polymer membranes. In some embodiments, the polymer membranes include nylon, polyamide, nitrocellulose, and PTFE. In some embodiments, the porous nanotextured substrate includes electro-deposited membranes. In some embodiments, the electro-deposited membranes include patterned metal and hydrogel matrices. In some embodiments, the porous nanotextured substrate includes anodized membranes. In some embodiments, the porous nanotextured substrate includes ceramic membranes. In some embodiments, the ceramic membranes can be made conformal or flexible when they are prepared as a mixture of alumina and silica combined in a ratiometric mixture and deposited and oxidized through chemical vapor or acid etching.

The conductive material may be any appropriate material known to those of skill in the art. In some embodiments, the conductive material is conductive ink or semi-conductive ink. In some embodiments, the semi-conductive ink comprises carbon ink and additives. In some embodiments, the conductive ink is carbon, silver, or metal nanoparticle-infused carbon inks. In some embodiments, the metal nanoparticle-infused carbon ink is infused with gold, platinum, tantalum, silver, copper, tin, or grapheme. In some embodiments, the carbon ink is infused with 0.1, 0.2, 0.3, 0.4, 0.5, 0.6, 0.7, 0.8, 0.9, 1, 2, 3, 4, 5, or more % by volume with the metal nanoparticles. In some embodiments, the thickness of the carbon ink ranges from 0.1 nm to 1 $\mu$m. In some embodiments, the thickness of the carbon ink may be controlled by the deposition method.

The circuit may be a nonlinear circuit or a non-ohmic circuit. In some embodiments, the circuit is further defined as a base electrode surface. In some embodiments, the base electrode surface is further connected to a source circuit. In some embodiments, the source circuit is a potentiostat. In some embodiments, the source circuit is a voltage source. In some embodiments, the source circuit is a current source. In some embodiments, the circuit does not contain a capture ligand or label-molecule. The conformal analyte sensor further comprises a redox material.

Any of the conformal analyst sensor circuits disclosed herein may be assembled by a method comprising (a) providing the solid porous nanotextured substrate; and (b) transferring the analyte sensor circuit design onto the top surface of the porous nanotextured substrate using conductive material. The assembly method does not form part of the claimed subject-matter. In some examples, transferring the circuit design comprises dip coating. In such examples, the feature resolution of the circuit is up to 100 nanometers/0.1 micron. In some examples, transferring the circuit design comprises embossing. In such examples, the feature resolution of the circuit is up to 100 nanometers/0.1 micron. In some examples, transferring the circuit design comprises designing the circuit on a 3D printer and embossing the circuit onto the substrate. In such examples, the feature resolution of the circuit is up to 100 nanometers/0.1 micron. In some examples, transferring the circuit design comprises masking and lithography. In such examples, the feature resolution of the circuit is 1-10 microns.

In some embodiments, the handheld potentiometer comprises an LCD screen, mini-joystick, working electrode port, reference electrode port, programmable microcontroller, and programmable gain amplifier. In other embodiments, the handheld potentiometer comprises a smartphone, cable, potentiostat adaptor, working electrode port, reference electrode port, programmable microcontroller, and programmable gain amplifier. In some embodiments, the handheld potentiometer comprises a programmable microprocessor instead of a programmable microcontroller.

In some embodiments, the handheld device for measuring a target analyte comprises (a) a programmable gain amplifier configured to be operably coupled to a working electrode and a reference electrode, (b) a programmable microcontroller operably coupled to the programmable gain amplifier, the working electrode, and the reference electrode, wherein the programmable microcontroller is operable to apply an alternating input electric voltage between the working electrode and the reference electrode; the programmable gain amplifier is operable to amplify an alternating output current flowing between the working electrode and the reference electrode; the programmable microcontroller is operable to calculate an impedance by comparing the input electric voltage to the measured output current; and the programmable microcontroller is operable to calculate a target analyte concentration from the calculated impedance.

In some embodiments, the programmable microcontroller is operable to apply an input electric voltage between the working electrode and the reference electrode that has a frequency between 2 Hz and 15 kHz. In some embodiments, the programmable microcontroller is operable to varying the frequencies between 50 Hz and 15 kHz in applying input electric voltages between the working electrode and reference electrode. In some embodiments, the programmable microcontroller varies the frequencies in 2 Hz intervals. In some embodiments, the programmable microcontroller is operable to apply an input electric voltage between the working electrode and the reference electrode that is sinusoidal.

In some embodiments, the programmable microcontroller is operable to apply an input electric voltage between the working electrode and the reference electrode that is a sawtooth wave. In some embodiments, the programmable microcontroller is operable to apply an input electric voltage between the working electrode and the reference electrode that is a square wave. In some embodiments, the programmable microcontroller is operable to apply an input electric voltage between the working electrode and the reference electrode that is a triangle wave. In some embodiments, the programmable gain amplifier has a variable gain of between 1 and 200. In some embodiments, the microcontroller is operable to apply an input electric voltage of between 1 mV and 10 V. In some embodiments, the handheld measuring device is operable to detect an output current of 10 pA or greater. In some embodiments, the programmable microcontroller comprises an analog to digital converter and a digital to analog converter. In some embodiments, the programmable microcontroller is capable of measuring a difference in phase between the input electric voltage and the output current. In some embodiments, the programmable microcontroller is operable to apply a Fourier transform to the input electric voltage and output current to calculate impedance as a function of frequency. In some embodiments, the programmable microcontroller is operable to use Lissajous curves to compare the input electric voltage and output current to calculate impedance. In some embodiments, the programmable microcontroller is operable to use multi-slice splitting and signal analysis to determine a frequency at which the impedance change is at a maximum or minimum. In some embodiments, the device further comprises a liquid crystal display operably coupled to the programmable microcontroller; a mini-joystick operably coupled to the programmable microcontroller; wherein the mini-joystick is operable to allow users to provide input; and the liquid crystal display is capable of displaying output data. In some embodiments, the device further comprises a smartphone operably coupled to the programmable microcontroller; wherein the smartphone is operable to allow users to provide input; and the smartphone is capable of displaying output data. In some embodiments, the output data comprises the target analyte concentration. In some embodiments, the handheld measuring device does not contain a redox probe.

Some examples, not forming part of the claimed invention, disclose a kit comprising any of the conformal analyst sensor circuits disclosed herein and any of the handheld measuring devices disclosed herein.

The handheld potentiostats and porous nanotextured conformal circuits disclosed herein may be used to detect and/or quantify a target analyte. In some embodiments, disclosed is a method of detecting a target analyte comprising spotting a sample on a disclosed conformal analyte sensor circuit, wherein the sample wicks through the porous nanotextured substrate and the circuit design, attaching the conformal analyte sensor circuit to a source circuit, and detecting the target analyte in the sample with a source circuit. In some embodiments, the source circuit is a potentiostat. In some embodiments, the source circuit is a voltage source. In some embodiments, the source circuit is a current source. In some embodiments, the sample contains 1, 2, 3, 4, 5, 6, 7 8, 9, 10, 11, 12, 13, 14, 15, or more $\mu$L of a fluid, or any amount in between. The sample may be, for example, blood, urine, sweat, saliva, lysis buffer, assay buffer, human serum, plasma, river water, stream water, and deionized water. In some embodiments, the target analyte is a protein, DNA, RNA, SNP, small molecules, pathogens, heavy metal ions, or physiological ions. In some embodiments, the sample is not labeled. In some embodiments, the sample is labeled. In some embodiments, detecting the target analyte comprises detecting an electrical change.

The claimed invention is a method of detecting or quantifying a target analyte in a sample using a handheld measuring device and a conformal analyte sensor circuit comprising the steps of (a) applying an input electric voltage between a reference electrode and a working electrode, (b) amplifying an output current flowing between the reference electrode and the working electrode using a programmable gain amplifier, (c) calculating an impedance by comparing the input electric voltage to the output current using a programmable microcontroller, and (d) calculating a target analyte concentration from the calculated impedance using a programmable microcontroller. In some embodiments, the input electric voltage has a frequency between 2 Hz and 15 kHz. In some embodiments, the input electric voltage has a frequency between 50 Hz and 15 kHz. In some embodiments, the input electric voltage is sinusoidal. In some embodiments, the input electric voltage is a sawtooth wave. In some embodiments, the input electric voltage is a square wave. In some embodiments, the input electric voltage is a triangle wave. In some embodiments, the input electric voltage is between 1 mV and 10 V. In some embodiments, the input electric voltage is between 1 mV and 100 mV. In some embodiments, the input electric voltage is between 100 mV and 10 V. In some embodiments, the output current is between 10 pA and 10 mA. In some embodiments, the output current is between 10 pA and 100 nA. In some embodiments, the output current is between 100 nA and 10 mA. In some embodiments, the output current is amplified by a factor between 1 and 200. In some embodiments, the method further comprises calculating a difference in phase between the input electric voltage and the output current. In some embodiments, the method further comprises calculating impedance as a function of frequency by applying a Fourier transform. In some embodiments, the method further comprises calculating impedance using Lissajous curves. In some embodiments, the method further comprises calculating impedance as a function of frequency using multi-slice splitting and signal analysis. In some embodiments, the method further comprises displaying the calculated target analyte concentration. In some embodiments, the method further comprises displaying an output on an LCD display. In some embodiments, the method further comprises displaying an output on a smartphone. In some embodiments, the method further comprises providing an input using a mini-joystick. In some embodiments, the method further comprises providing an input using a smartphone. In some embodiments, the measured impedance is non-faradaic.

The handheld potentiometer detects concentrations of a target analyte by applying an alternating voltage between the working and reference electrodes. The applied alternating voltage results in a current flowing between the working and reference electrodes. The resulting current is amplified by a programmable amplifier and passed onto the programmable microcontroller. The programmable microcontroller compares the applied voltage to the resulting current to calculate the impedance of the tested sample. The impedance is used to calculate the concentration of the target analyte in the tested sample. In some examples, not part of the claimed invention, to perform testing of a target analyte using the handheld potentiometer, the handheld potentiometer is first calibrated by testing and calculating the impedance of samples containing known quantities of the target analyte. In some embodiments, the system applies voltages of varying frequencies and determines the frequency at which the maximum impedance change occurs for a particular tested analyte. The claimed system may perform non-Faradaic electrochemical impedance spectroscopy ("EIS") by testing samples without using a redox electrode.

In some examples, not part of the claimed invention, disclosed herein is a method of calibrating a handheld measuring device by testing a plurality of solutions having known target analyte concentrations comprising (a) applying an input electric voltage between a reference electrode and a working electrode for each of the plurality of solutions, (b) calculating an impedance for each of the plurality of solutions by comparing the input electric voltage to the output current using a programmable microcontroller, and (c) calculating coefficients of the equation $z_i= b_1x^2+ b_2x+c$, wherein $z_i$ is the impedance, x is the known target analyte concentrations, and $b_1$, $b_2$, and c are the coefficients.

As used herein the specification, "a" or "an" may mean one or more. As used herein in the claim(s), when used in conjunction with the word "comprising", the words "a" or "an" may mean one or more than one.

The use of the term "or" in the claims is used to mean "and/or" unless explicitly indicated to refer to alternatives only or the alternatives are mutually exclusive, although the disclosure supports a definition that refers to only alternatives and "and/or." As used herein "another" may mean at least a second or more.

Throughout this application, the term "about" is used to indicate that a value includes the inherent variation of error for the device, the method being employed to determine the value, or the variation that exists among the study subjects.

Other objects, features and advantages of the present invention will become apparent from the following detailed description. It should be understood, however, that the detailed description and the specific examples, while indicating preferred embodiments of the invention, are given by way of illustration only, and that the scope of the invention is defined by the appended claims.

## BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.

**FIG. 1** High resolution optical micrograph demonstrating the surface porosity and interaction between the pores and the electrode surfaces, including a scanning electron micrograph showing conformal feature generation between the electrode and the surrounding matrix with a schematic rendering of the interaction between the measurement entity and the surrounding matrix.

**FIG. 2** Assay demonstration in the impedance format for detecting Troponin-T in human serum.

**FIG. 3** Assay demonstration in the impedance format for detecting atrazine in drinking water.

**FIG. 4** Gating characteristics of the conformal circuit in DNA diagnostics.

**FIG. 5** A schematic representation of a representative two electrode handheld potentiostat.

**FIG. 6** Assay demonstration in the impedance format comparing the performance of the present invention versus the Roche Elecsys in detecting Troponin-T in human plasma.

**FIG. 7** Assay demonstration in the impedance format for detecting PSA in human serum.

**FIG. 8** Handheld potentiostat device.

**FIG. 9** A flow chart demonstrating the operation of a potentiostat.

**FIG. 10** A smartphone embodiment of a handheld potentiostat.

**FIG. 11** A flow chart demonstrating the impedance and analyte concentration calculations performed by a potentiostat.

**FIG. 12** A sample Lissajous curve.

## DESCRIPTION OF ILLUSTRATIVE EMBODIMENTS

The conformal circuits disclosed herein leverage the surface roughness that exists at the nanoscale on paper and other nanoporous substrates for designing conformal electric circuits. Circuit parameters such as current and impedance are modulated when the circuit elements are modulated due to the detection of biomolecules through a single step immunoassay format. This technology can be applied towards detecting and quantifying a variety of target analytes, including but not limited to proteins, DNA, RNA, SNP, and a diverse range of biomolecules.

Disclosed herein are conformal circuits comprising a solid substrate having a top surface, wherein the substrate comprises porous nanotextured substrate and a conductive material situated on the top surface of the solid substrate in a circuit design, thereby creating a circuit. Also disclosed are methods of making the same, as well as methods of detecting and/or quantifying a variety of target analytes using the same. FIG. 1 depicts an example design of such a conformal circuit.

These conformal circuits are developed using a combination of track etching and conductive ink deposition to create nonlinear and non-ohmic circuits. Three types of circuits are generated: (a) impedance-based resistive capacitive (RC) coupled circuits, (b) two-terminal non-linear device-based circuits, and (c) non-linear device-based circuits. The RC circuits work on the principle of electrochemical impedance spectroscopy, and the two-terminal non-linear device and non-linear device circuits are biased by an AC voltage source resulting in changes to current characteristics as a function of detection of species of interest.

The conformal circuits disclosed herein have two electrodes that are conducting. An increase in conductivity is suitable for achieving increased sensitivity in the impedance measurement format. In preferred embodiments, an AC voltage between 1 mV and 10 V will be applied to the electrodes. In preferred embodiments, an AC voltage having a frequency that varies between 2 Hz and 15 kHz will be applied to the electrodes.

The conformal circuits disclosed herein generate electrical changes as opposed to electrochemical changes. In particular, the conformal circuits disclosed herein generate electrical/electrochemical changes without the use of a reduction-oxidation probe, as opposed to electrochemical changes mediated through a redox electrode. The use of a redox probe for electrochemical detection produces irreversible changes to the biomolecule resulting in indirect and modified detection that is not representative of the biomolecules. The capability of generating electrical/electrochemical changes without the use of a reduction-oxidation probe is achieved by tailoring the deposition of the conductive material onto the nanoporous substrate. In addition, both passive and active sensing are specifically contemplated.

The conformal circuit and detection devices disclosed herein can be designed to detect quantitatively (e.g., an EIS electronic reader). In addition, the system can be designed to detect a single analyte using a single circuit or multiple analytes using separate circuits, which may be the same or different, depending on the variety of analytes being detected and/or analyzed.

### A. Substrates and Conductive Materials

The substrates contemplated include porous nanotextured substrates. In some embodiments, the use of paper, nitrocellulose, fabric, leaves, bark, or shells is contemplated; however, any porous, hydrophilic substrate that wicks fluids by capillary action can be used as the substrate in the methods and devices described herein. Non-limiting examples include cellulose and cellulose acetate, paper (e.g., filter paper and chromatography paper), cloth or fabric, porous polymer film, porous plastic, or leaves. In some embodiments, the substrate is biodegradable. In some embodiments, the substrate is paper. Any naturally occurring substance with flexibility and thickness under 500 $\mu$m can serve as the substrate so long as the degradation temperature of the naturally occurring substance is higher than the temperature of deposition.

In some embodiments, the substrate includes a hydrophobic coating, such as parylene, polyamide, PEG, polycation solutions, and polydimethlysiloxane. The hydrophobic coating is used to isolate and contain the fluid on the active sensor substrate. In some embodiments, the substrate includes surface coatings, such as pre-formulated sprays and aerosols, that are biocompatible and can introduce hydrophobicity on specific regions of the substrate. Examples of surface coatings include a mixture of ethanol, polydimethlysiloxane, ethyl sulfate, chlorotrimethylsilane, siloxanes and silicones as well as pre-formulated block co-polymer mixtures. In some embodiments, the substrate includes track etched, acid etched, anodized, polymer, ceramic, and electro-deposited membranes. Ceramic membranes can be made conformal or flexible when they are prepared as a mixture of alumina and silica combined in a ratiometric mixture and deposited and oxidized through chemical vapor or acid etching. Examples of track etched membranes include nucleopore and cyclopore form factors. Examples of acid etched membranes include silicon and alumina scaffolds. Examples of polymer membranes include nylon, polyamide, nitrocellulose, and PTFE. Examples of electro-deposited membranes include patterned metal and hydrogel matrices.

The porosity of the substrate in conjunction with conductive ink screen printing can be leveraged to pattern conformal circuits. Any size and thickness of substrate may be used, as the dimensions of the substrate are not key to functionality of the circuit. The critical parameter that impacts the performance of the circuit is the porosity of the substrate. Porosity can vary from 10 x $10^5$ to 10 x $10^{20}$ pores/$cm^2$, and the substrate, including its porosity, is selected based on the size of the target analyte. This porosity can be adjusted or tuned using a variety of techniques, e.g., coatings or treatments. The pore size may vary from 1 nm to 200 nm. Pore size is defined for the application based on the size of target analyte and frequency of applied electrical signal. Pore-to-pore spacing is always greater than average pore size on the membrane substrate. Examples of possible treatments and coatings include wet treatments such as acidic or alkaline etching, use of layer by layer deposition of self-assembled monolayers, and dry treatments such as reactive ion etching and plasma etching.

The substrate can be up to 500 μm thick and there are no capping factors on the lateral dimensions. In some embodiments, the substrate may be 1, 2, 3, 4, 5, 6, 7, 8, 9, or 10 cm by 1, 2, 3, 4, 5, 6, 7, 8, 9, or 10 cm, or any size in between. In particular embodiments, the substrate is 1 cm by 1 cm.

It is contemplated that any appropriate conductive material may be used as the conductive ink and a range of conductive inks are contemplated. Conductive inks usually contain conductive materials such as powdered or flaked silver and carbon like materials. In some embodiments, the conductive ink is carbon, silver, or metal nanoparticle-infused carbon inks. Non-low melt gallium deposited under a vacuum used a heated chuck and target can be used and followed with low melt gallium ink alloying. In some embodiments, the metal nanoparticle-infused carbon ink is infused with a noble metal. In certain examples, the carbon ink is infused with gold, platinum, tantalum, silver, copper, tin, or grapheme. The use of additives such as metal nanoparticles to carbon ink changes the conductive carbon ink into semi-conducting ink. In some embodiments, the carbon ink is infused with 0.1, 0.2, 0.3, 0.4, 0.5, 0.6, 0.7, 0.8, 0.9, 1, 2, 3, 4, 5, or more % by volume with the metal nanoparticles. In some embodiments, the thickness of the carbon ink varies from 0.1 nm to 1 μm. The thickness of the carbon ink is controlled with deposition methods. In some embodiments, this semi-conducting ink pattern may be used for designing the two-terminal non-linear device and non-linear device behavior. In some embodiments, native conducting ink may be used for obtaining impedance changes. The ink substrate (i.e., the combination of the ink and the substrate) is the base electrode surface over which the biomolecule chemistry is implemented for achieving molecular diagnostics.

The nature of the ink is dependent on the type of sensing and analysis desired. In some embodiments, when passive sensing with an electrical reader is necessary, the ink is only conducting. More particularly, for passive devices, conductive/semi-conducting nanoparticles may be dispersed in a matrix or the ink may contain metal nanoparticles or electro active polymer matrices. In situations where active sensing, such as where a multimeter or potentiostat is used, the ink can be conducting and semi-conducting, or conducting stacks.

The conformal circuit includes a redox material, such as derivatives of copper, potassium, magnesium, and rubidium. These materials bind with the receptor of the analyte immobilized onto the conformal circuit. During the binding of the analyte onto the receptor with the redox material there is an amplification in the number of charges routed through the conformal circuit due to the reduction or oxidation of the redox material. This process is distinct from the use of redox electrodes, where the redox material is immobilized onto the redox electrode itself. In that process, during the application of a bias potential or a current to the redox material on a redox electrode, this material undergoes either a reduction or oxidation, thus binding to the target analyte in this state and modifying the analyte that is being tested/evaluated.

**B. Methods of Patterning**

The conformal circuits are assembled by performing engineering to standard paper products. Porosity in paper is leveraged towards achieving control in circuit formation. A stencil of the circuit design is transferred onto the substrate surface in any appropriate manner. The parameters of the desired pattern are determined by the molecules to be detected. A person of skill in the art would recognize the appropriate transferring method in view of the desired pattern. For example, smaller patterns or smaller feature sizes require the more advanced printing techniques, e.g., masking and lithography. These processes are discussed in more detail below.

Stencils contain a pattern of holes or apertures through which conductive materials could be deposited onto the hydrophilic substrates. Alternatively, in an etching process, stencils contain a pattern of holes or apertures through which conductive materials could be etched to form a pattern of metal on the hydrophilic substrates. Stencils could be made from a variety of materials such as metal, plastic, or patterned layers of dry-film resist. Non-limiting examples of metals for manufacturing stencils include stainless steel and aluminum. Non-limiting examples of plastic for manufacturing stencils include mylar. Alternatively, patterned layers of dry-film resist can be used as stencils. In one or more embodiment, metals or plastics are used to manufacture stencils and patterns of metallic pathways can be designed on a computer using a layout editor, (e.g., Clewin, WieWeb Inc.) and stencils based on the design can be obtained from any supplier (e.g., Stencils Unlimited LLC (Lake Oswego, Oreg.)). In certain embodiments, the stencil can be removed from the paper after deposition. In certain other embodiments, one side of the stencil is sprayed with a layer of spray-adhesive (e.g., 3M Photomount, 3M Inc.) to temporarily affix the stencil to the paper substrate. After deposition, the stencil can be peeled away from the paper. The stencils can be reused multiple times, e.g., more than ten times. In other embodiments, patterned layers of dry-film resist can be used as stencils. Dry film resist can be patterned when exposed to UV light through a transparency mask and developed in dilute sodium hydroxide solution. The patterned dry-film resist can be attached to a coating sheet of plastic or directly affixed to the hydrophilic substrates by pressing the resist-side to the surface of the hydrophilic substrates and passing multi-sheet structure through heated rollers in a portable laminator (e.g., Micro-Mark, Inc.). The coating sheet of plastic can then be peeled away, resulting in a sheet of paper with dry film resist patterned on one side.

A variety of deposition methods could be used to deposit electrically conductive materials onto the hydrophilic substrates of the microfluidic devices. Non-limiting examples of the deposition methods include depositing conductive materials using stencils, depositing conductive materials by drawing conductive pathways, depositing conductive materials by inkjet or laser printing, depositing conductive materials by attaching commercially available or homemade conductive material tapes onto the hydrophilic substrates, depositing conductive materials by drawing conductive pathways, or depositing conductive materials by introducing conductive fluids onto the hydrophilic substrates or the hydrophilic channels of the microfluidic devices. Alternatively, conductive materials could be embedded in the pulp or fibers for manufacturing the hydrophilic substrates to allow for manufacturing hydrophilic substrates containing conductive materials.

It is specifically contemplated that the circuit design may be transferred onto the substrate surface either through (a) dip coating, (b) embossing, or (c) masking and lithography. Dip coating and embossing allow for feature resolution which is greater than 1 micron, and masking and lithography allows for feature resolution in 1-10 micron regime. These techniques are well known to those of skill in the art. See Reighard and Barendt, 2000. In particular embodiments, the circuit may be designed on a 3D printer and the design may be transferred to the substrate by embossing the circuit onto the substrate.

The lateral porosity of the substrate is leveraged to generate the conformal circuits disclosed herein. Vertical porosity is not suitable, and therefore in some embodiments a metal barrier of thickness in the order of 100s of nm achieves this goal. The thickness of deposited material also corresponds to the thickness of the substrate in some regions to change the electrical behavior of the substrate. Lateral porosity helps in enabling flexibility to the metal electrodes patterned which in turn enables the conformal physical nature of the substrate. The deposited material can be used to support the metal electrodes and increase or reduce conductivity without compromising on the conformal physical nature of the substrate.

In some examples , the entire paper surface is dip coated. Biomolecules interacting with the conductive ink surfaces alone are responsible for the measured signal. There are no flow considerations to be taken into account. Hence, biomolecule interactions are primarily diffusion and capillary action driven and therefore larger the pores, the faster are the interactions. Multiple layers of dip coating have been adopted, where appropriate. This technique is most relevant when the intent is to design immunoassays requiring multiple layers of molecules incorporated onto the sensor platform.

**C. Detection of Biomolecules**

These conformal circuits can be applied for a wide range of molecular diagnostics and analysis, and therefore can be used on any sample that is suspected of containing a molecule of interest such as food, water, soil, air, bodily fluids such as blood serum, detergents, ionic buffer, etc. In some embodiments, the sample is any liquid sample or solid that can be solubilized or dispersed in a liquid. The circuits can be used to design simple affinity based assays for mapping presence of enzymes and physiological ions. These can be used to develop assays to study antibody-antigen interactions and to determine presence or absence of a wide range of protein biomarkers expressed at ultra-sensitive concentrations. Genomic assays can also be developed using these circuits.

A single step immunoassay can be used in connection with the conformal circuits. In some embodiments, label free immunoassays using electrochemical sensors are appropriate (Vertergaard, *et al.,* 2007). In a particular embodiment of protein diagnostics, a single primary antibody without a tag is used and, based on the base circuit, controlled and mapped modulations to the electrical circuit parameters are achieved during detection of the proteins. The system can be designed to detect quantitatively (e.g., an electrochemical impedance spectroscopy electronic reader).

The conformal circuits disclosed herein may be prepared for the immunoassay in any appropriate manner. In one embodiment, a linker is deposited on the substrate, the substrate is saturated with a moiety specific for the target analyte, e.g., a target specific antibody, a blocking buffer is applied to the receptor moiety saturated conformal circuit surface to minimize nonspecific binding or adsorption of other competing molecules onto the sensor surface, a buffer wash is performed, and the target analyte, e.g., antigen, is dosed onto the circuit. In designing the calibration curve for a target molecule, such as an antigen, increasing doses of the antigen are applied onto the conformal circuit and impedance measurements are obtained until steady state is reached. An increasing change to the measured impedance is expected with increasing dose of the target molecule such as an antigen. Once the calibration curve has been designed, an unknown dose of a test target molecule such as an antigen is tested onto the antibody/receptor moiety saturated sensor surface, and the change in impedance is then evaluated against the calibration curve to determine the dose of the test target molecule.

Analyte confinement is achieved within the nanoscale texture of the substrate, and the size-based confinement of the target analyte onto the substrate is achieved using conductive ink. Analytes interacting with the conductive ink in a single step immunoassay format perturb the (a) electrical double layer, (b) charges in the depletion layer in the two-terminal non-linear device, and (c) gate current characteristics of non-linear device resulting in the detection of the biomolecule of interest. As ultra-low volumes in the range of 1-10 micro liters are generally used, the issue of controlled flow does not exist. Primarily spotting of the fluid on the substrate surface is sufficient to achieve associated interaction for biomolecule detection.

For a single channel assay, a sample volume of less than 125 $\mu$L is needed, it has a dynamic range of detection of 0.1 pg/mL - 10 $\mu$g/mL, and it can be useful for molecules at or between 1 nm and 1 $\mu$m.

**D. Detection Devices**

A variety of electrical components can be attached to the electrically conductive material pathways in order to detect and quantify the target analyte. Non-limiting examples of electronic components include integrated circuits, resistors, capacitors, transistors, diodes, mechanical switches, batteries, and external power sources, non-limiting examples of batteries include button cell batteries, and non-limiting examples of external power sources include an AC voltage source. The electrical components can be attached using, e.g., known adhesives. The conformal circuits discussed in detail above are coupled to a handheld measuring device for the purpose of detecting the biomolecule. In particular embodiments, the conformal circuit can be coupled to potentiostats, voltage sources, current sources, or operational amplifier circuits for doing a wide range of simple and complex mathematical operations, addition, subtraction, integration, and differentiation.

Impedance spectroscopy is a widely used two or three electrode electrochemical technique for studying material binding efficiency on electrodes. Recently, innovative changes to classical electrochemical impedance spectroscopy have made it suitable for applications to biomedical studies. These modifications demand application of very low voltages and detection at very small currents, both of which fall into the noise threshold of existing devices. In addition, most currently available market potentiostats require additional equipment, such as a computer, and detailed user input, making it difficult for point-of-care implementation.

Disclosed herein are customizable handheld potentiostats devices for performing electrochemical impedance spectroscopy using a two electrode configuration at fixed and variable frequencies. The novel technique used in the disclosed device reduces noise effects and achieves sensitive detection, and the components used are programmable and highly customizable to the desired application. Consequently, this achieves maximum performance efficiency from the device by programming it to function best in the desired range of operation for the particular desired task.

In the devices disclosed herein, impedance spectroscopy is used to detect and quantify binding activity on an electrode surface. The binding of biomolecules to an electrode surface causes a change in current flow, which can be used to detect or quantify the biomolecule being bound. The detection threshold for the device is approximately 0.1 pg/mL.

In the devices disclosed herein, Helmholtz probing is used. Helmholtz probing is a technique with the ability to section the electrical double layer into sections/planes and study it in a spatio-temporal manner. Specific changes to capacitance and impedance in a section/plane can be used to detect specific binding of targets to capture probes.

The handheld potentiostats disclosed herein are made up of a working and reference electrode. An AC voltage is applied at the working and reference electrode terminals. The AC voltage may be a sinusoidal, sawtooth, square, or triangle wave signal. The resulting current flowing between the working and reference electrode terminals is then measured.

A diagram depicting an example of one configuration of a handheld potentiostat is found at FIG. 8. The handheld potentiostat 200 comprises an LCD display 104. The LCD display 104 provides a user interface that displays input and output data. For example, the LCD display may show an input voltage, an input frequency, a wave type, and a molecular concentration. The handheld potentiostat 200 may also comprise a mini-joystick 110, which enables the user to provide input to the handheld potentiostat 200. For example, the mini-joystick 110 may be used to navigate menus on the LCD display 104 and increase or decrease voltage and frequency values. In some embodiments, the handheld potentiostat 200 may comprise buttons or a keypad in addition to or instead of a mini-joystick 110. The handheld potentiostat further comprises a working electrode port 202 and a reference electrode port 204. The electrode ports 202 and 204 are used to connect wire leads to the working and reference electrodes.

A block diagram representing one possible potentiostat / electrode configuration is found at FIG. 5. The heart of operation for the potentiostat is carried out in the programmable microcontroller/microprocessor 100. The first operation of the microcontroller is providing user interface support through an LCD display 104. The serial peripheral interface 138 is used to communicate information processed in the microcontroller 100 to the LCD display 104. The microcontroller 100 uses lines 134 and 136 to supply power to the LCD display 104.

User input/response to options displayed on the LCD display 104 is received as analog signals through an analog-analog communication between the mini-joystick 110 and microcontroller 100. Using the mini-joystick 110, the user may select the electrical signal parameters, e.g., voltage, frequency, wave type, to be applied to the working electrode 106 and reference electrode 108. Alternatively, the mini-joystick 110 is used to select the type of molecule to be detected. After the test concludes, the LCD display 104 shows the numerical concentration of the molecule in the tested sample.

Next, the microcontroller 100 is programmed to perform impedance spectroscopy characterization on the attached electrochemical sensor. Based upon the electrical signal parameters or molecule selected by the user, the programmable microcontroller 100 generates an AC voltage on lines 130 and 132 that is applied to the working electrode 106 and reference electrode 108, respectively. The AC voltage may be amplified by amplifiers 112 and 114. In some embodiments, the resulting voltage of the working electrode 106 may fed back to the microcontroller 100 on line 140. The resulting voltage may differ from the applied voltage due to chemical reactions in the tested solution. The microcontroller 100 digitizes the voltage value of the working electrode 106, and the digitized voltage is used by the microcontroller 100 to adjust the applied AC voltage level on lines 130 and 132. In some embodiments, the voltage of the working electrode 106 may fed back to the programmable gain amplifier 102 on line 122. The programmable gain amplifier may digitize the voltage value of the working electrode 106 and send the digitized voltage to the microcontroller 100 over line 128, and the digitized voltage is used by the microcontroller 100 to adjust the AC voltage level on lines 130 and 132.

After an AC voltage is applied and a sample of an electrically conductive solution contacts the sensor, an AC current flows from the working electrode 106 to the reference electrode 108. The amount of current flowing through the working electrode 106 and reference electrode 108 depends upon the voltage applied to the working and reference electrodes, the binding of molecules on the electrodes, and the solution used. A programmable gain amplifier 102 measures the current flowing between the working electrode 106 and reference electrode 108. Specifically, the transconductance amplifier 116 feeds a current to the programmable gain amplifier on line 124. The current may be filtered by a bandpass filter 120. The bandpass filter 120 is automatically adjusted to permit signals at the applied frequency while rejecting noise at other frequencies. The programmable gain amplifier 102 then generates an amplified voltage from the current that is fed into the programmable microcontroller on line 126. The amplification is necessary as the microcontroller operation thresholds are much greater than the small voltages and currents generated in this impedance spectroscopy application. In some embodiments, the amplified voltage on line 126 ranges between 20 mV and 6 V. If the amplified voltage on line 126 is too high or too low, the microcontroller 100 sends a signal to the programmable gain amplifier 102 over line 128 to increase or decrease the gain. In some embodiments, the binary gain of the programmable gain amplifier 102 may be adjusted between 1 and 128. In some embodiments, the scope gain of the programmable gain amplifier 102 may be adjusted between 1 and 200. Line 122 provides a reference voltage to the programmable gain amplifier 102 to calculate gain. Line 122's voltage may be amplified by amplifier 118 and filtered by a bandpass filter 120.

The microcontroller 100 converts the analog amplified voltage to a digital signal. The microcontroller 100 then compares the digitized amplified voltage, which represents the amount of current flowing between working electrode 106 and reference electrode 108, to the voltage applied to the working electrode 106 and reference electrode 108 to determine the impedance of the solution being tested. The microcontroller 100 performs arithmetic operations to calculate phase and amplitude changes in the amplified voltage with respect to the applied voltage as a function of frequency. Impedance is calculated using the following formula:

$$Z = \frac{V_m \sin \omega t}{I_m \sin(\omega t + \varphi)}$$

where $V_m$ represents the amplitude of the applied voltage, $I_m$ represents the amplitude of the resulting current flowing between the electrodes, co is the angular frequency of the applied voltage and resulting current, and $\varphi$ is the difference in phase between the applied voltage and resulting current. In some embodiments, the microcontroller 100 uses a Fourier transform to determine the phase and amplitude changes as a function of frequency. In some embodiments, the microcontroller 100 uses Lissajous curves to determine the phase and amplitude changes. In some embodiments, the microcontroller 100 performs multi-slice splitting and signal analysis to determine at which frequencies the change in impedance is the greatest. This estimation helps in characterizing the bio-electrochemical reactions occurring on the surface of the electrodes. The microcontroller 100 uses the change in amplitude and phase to calculate the concentration of the molecule in the sample.

Before being used to measure unknown quantities of a target analyte, the handheld potentiostat may be calibrated. Calibration is performed by measuring the impedance of solutions containing known quantities of a target analyte. Specifically, the user may perform impedance measurements of preferably four different solutions containing four different concentrations of the target analyte. For each calibration test, the user inputs the target analyte concentration into the handheld potentiostat using the mini-joystick. The handheld potentiostat records the impedance for each test. After the tests are completed, the system completes the calibration by determining the coefficients in the following equation,

$$z_i = b_n x^n + b_{n-1} x^{n-1} + \ldots + b_1 x + c$$

where $z_i$ is the measured impedance, x is the known concentration of the target analyte, and $b_n$, $b_{n-1}$, $b_1$, and c are the coefficients. The order of the polynomial, n, may be between two and five, and preferably two. The handheld potentiostat determines the unknown values of the coefficients using linear regression and least squares analysis.

A flowchart depicting the detection of molecules using a handheld potentiostat is found at FIG. 9. At step 400, the user provides input, such as the choice of voltage or frequency, regarding the electrical signal that the handheld potentiostat will apply to the sample. At step 402, the microcontroller applies an electrical signal to the working electrode and reference electrode. The characteristics of the electrical signal, e.g., voltage and frequency, are based upon the inputs provided by the user in step 400. At step 404, the microcontroller receives a reference signal from the working electrode. Specifically, the working electrode voltage is amplified by a gain amplifier, and the amplified voltage is fed into the microcontroller's digital-to-analog converter ("DAC"), which converts the analog amplified voltage into a digital signal. In some embodiments, a programmable gain amplifier converts the analog amplified voltage into a digital signal. The microcontroller compares the value of the digital working electrode voltage to the desired voltage selected by the user. The microcontroller may then increase or decrease the applied electrical signal in step 402 to match the desired voltage that was selected in step 400. At step 406, the working electrode voltage value is fed into a gain amplifier and converted into a current. At step 408, a gain amplifier in the programmable gain amplifier amplifies the current signal and converts the current signal into a voltage signal. The voltage signal then enters the micrcontroller's ADC. At step 410, the microcontroller converts the analog voltage signal into a digital voltage signal. At step 412, the microcontroller compares the digital voltage signal to calibration data stored in the memory of the microcontroller. In some embodiments, the microcontroller compares the measures the analog voltage signal to stored calibration data. In some embodiments, the microcontroller compares the digital voltage signal to a calibration data to determine a difference in amplitude and phase as a function of frequency. In some embodiments, the microcontroller compares the digital voltage signal to a calibration data to determine a difference in amplitude and phase as a function of frequency. The choice of method depends on the noise level of the signal. Fourier transform is best used when noise signals are very high in the transmission lines.

In some embodiments, the microcontroller 100 is an Intel® microcontroller. In other embodiments, the microcontroller 100 is an Intel® microprocessor. In other embodiments, the microcontroller 100 is an ARM Cortex™-M microcontroller. In other embodiments, the microcontroller 100 is an ARM Cortex™ microprocessor.

In preferred embodiments, the microcontroller 100 applies an AC voltage between 1 mV and 10 V to the working electrode 106 and the reference electrode 108. The microcontroller applies an AC voltage whose frequency varies between 2 Hz and 15 kHz to the working electrode 106 and the reference electrode 108. The frequency is varied by increasing from a minimum to a maximum frequency or decreasing from a maximum to a minimum frequency. In some embodiments, the user selects a minimum and a maximum frequency, and the microcontroller 100 applies voltages having frequencies that vary between the selected minimum and maximum frequencies. In some embodiments, the microcontroller 100 varies the frequency between the minimum and maximum frequencies in 2 Hz intervals.

In some embodiments, the handheld potentiostats disclosed herein perform impedance spectroscopy analysis on a biosensing platform. Very low voltage is necessary for the use of these potentiostats in order to be applicable for biosensing, as proteins and biomolecules are sensitive. In some embodiments, the range of appropriate voltage may be may be 1 mV to 10 V, but the appropriate voltage will depend on the application. In applications to protein based sensing, the voltages will be in the range of 1 mV to 10 V. In application to cells and DNA, the voltage ranges will be between 1 mV to 10 V. Similarly, due to the application of very small voltages, the current response is in a similar range or much lower, as there is loss due to bulk solution medium. In some embodiments, the range of appropriate current is 10 pA to 10 mA and, as with the voltage, the appropriate current response will depend on the application. In applications to protein based sensing, the current response will be in the range of 10 pA to 100 nA. In application to cells and DNA, the current response will be between 100 nA to 10 mA. The power required by the handheld potentiostat will be in the range of 2 mW to 10 W. The power level varies based on the casing, volume, time, analyte size, and the detection of single or multiple analytes.

The disclosed potentiostats may be used at fixed or variable frequencies. Based on the application, the fixed and variable frequency ranges will vary. For most biosensing applications, the range of frequencies used is between 2 Hz and 15 kHz. Upon optimization of the electrical debye length changes corresponding to a protein of interest, the fixed frequency can be estimated. Detection at the respective frequency can improve detection speeds and reduce non-specific signals.

In addition to performing impedance spectroscopy, the handheld potentiostats disclosed herein can be used as a source meter and also as a voltammetry tool through easy-to-choose options on the LCD display.

The handheld potentiostats disclosed herein are easily portable and have a hand friendly form factor. It may be about or at least 1, 2, 3, 4, 5, 6, 7, 8, 9, or 10 inches by about or at least 1, 2, 3, 4, 5, 6, 7, 8, 9, or 10 inches. It is specifically contemplated that it may be about 5 inches by about 3 inches. It is also specifically contemplated that the entire device, including the programmable gain amplifier, the programmable microcontroller, and the LCD display for output that are indicated on the diagram, be within these sizes.

A diagram depicting a smartphone embodiment of the handheld potentiostat is found at FIG. 10. The handheld potentiostat comprises a smartphone 300 and a potentiostat adaptor 306. The smartphone is operably coupled to a potentiostat adaptor 306 using a cable 304, preferably a Micro USB or a proprietary connector. The cable 304 provides bidirectional communication between the smartphone 300 and the potentiostat adaptor 306. The potentiostat adaptor comprises a working electrode port 202, a reference electrode port 204, a microcontroller 100, and a programmable gain amplifier 102. Users install a custom potentiostat software application onto the smartphone 300 that provides user input and output and microcontroller communication functionality. Users may provide input to the smartphone 300, including the input voltage, input frequency, and wave type, using a touchscreen 302. In other embodiments, users provide input to the smartphone using a keypad. The smartphone 300 displays output, such as the concentration of the target analyte on the smartphone's touchscreen 302.

The potentiostats disclosed herein also perform with low noise threshold at the desired range of operation for biosensing. Currently, potentiostats are designed with electrochemical applications in mind. The integrated circuits used for these applications have reasonable noise thresholds. When applying to biosensing, the measured signals of the available devices are in many cases within the noise threshold, thus rendering majority of the available potentiostats unsuitable.

The potentiostats disclosed herein are also programmable to perform two electrode impedance spectroscopy using Fourier transforms and Lissajous curve method. Existing potentiostats use Lissajous curves methods to estimate phase change in the measured current response. Though this has been perfected for applications involving high voltages and currents, it is not optimized for analysis of voltage and current responses as necessary for biosensing. Fourier transform-based estimation, which is more appropriate for these applications, has not been widely used due to complexity in implementation as it demands high processor speeds. Using Lissajous curves and Fourier transforms assists in digital signal analysis by reducing noise and preserving signal integrity; both of which are critical for biosensing.

A flowchart demonstrating the potentiostat's calculations using Fourier transforms and Lissajous curves is shown at Fig. 11. At step 500, the microcontroller applies a sinusoidal voltage of the form $V(t) = v \sin(\omega t)$, where v is the amplitude of the signal and co is the angular frequency. In preferred embodiments, the microcontroller applies sinusoidal voltages at varying frequencies. At step 502, the microcontroller measures the resulting current signal, which is of the form $I(t) = i \sin(\omega t + \varphi)$, where i is the amplitude of the signal and φ is the phase shift of the signal. The microcontroller converts the applied voltage signal from the time domain into the frequency domain in step 504 by applying a Fourier transform, $V(\omega) = \frac{1}{T}\int v(t)e^{j\omega t}\,dt$. Likewise, the microcontroller converts the resulting current signal from the time domain into the frequency domain in step 506 by applying a Fourier transform, $I(\omega) = \frac{1}{T}\int i(t)e^{j\omega t}\,dt$. At step 508, the resulting current frequency signal is verified with the applied voltage signal and noise occurring at other frequencies is filtered out. As an alternative to steps 504-510, the microcontroller plots Lissajous curves of v(t) and i(t) to estimate the impedance Z(t) at step 512. Fig. 12 illustrates a sample Lissajous curve, where E represents the applied voltage and I represents the resulting current. In this example, the applied voltage is a sinusoidal wave that varies between 15 and -15 mV, and the resulting current varies between 45 and 55 nA. The intersection of the voltage and current on the plot in this example is an ellipse, which indicates that the system is stable. Analysis of the elliptical region provides an estimate of the resulting impedance. At step 514, the microcontroller coverts impedance to the frequency domain using the equation $Z(\omega) = \frac{1}{T}\int Z(t)e^{j\omega t}\,dt$. At step 516, the microcontroller calculates the change in impedance, $\Delta Z(\omega)$, using the formula $\Delta Z(\omega) = Z_b(\omega) - Z(\omega)$, where $Z_b(\omega)$ is the impedance of the control sample. At step 518, the microcontroller determines the frequency at which the maximum impedance change occurred using multi-slice splitting, wherein the applied frequency spectrum is sliced into individual discrete frequency points. The microcontroller then compares the frequency at which the maximum impedance change occurred to the reference frequency point stored in memory for the specific analyte being tested at step 520. At step 522, the microcontroller estimates the concentration of the tested analyte by applying the same equation used in calibration, $z_i = b_n x^n + b_{n-1} x^{n-1} + \ldots + b_1 x + c$, where $z_i$ is the impedance at the frequency at which the maximum impedance change occurred, and $b_n$, $b_{n-1}$, $b_1$, and c are coefficients calculated during calibration, and x is the target analyte concentration being computed. In preferred embodiments, the equation in step 522 is quadratic. Step 524 illustrates the change in impedance as a function of target analyte concentration.

The potentiostats disclosed herein also contain cost-effective components, manufacturing involves very simple surface mount device assembly, and the disclosed devices have low-thermal noise due to use of modern current amplifiers and programmable gate arrays.

Finally, the potentiostats disclosed herein have applicability as a source meter, a voltammetry tool, and for standard current measurements. The potentiostats can be customized for the different applications by making modifications to the program that run the operations and produce results. The programmable gain amplifiers have a broad range of operation (mV-V/pA-mA) and hence can be used for different voltammetry applications to biosensing as well as general applications. Microprocessors/microcontrollers offer extensive programming liberties and hence application of the potentiostats to different operations will require only software changes and not hardware.

The potentiostats disclosed herein are highly adaptable and generates results rapidly. For a single channel assay, when a single channel EIS detection scheme and a 16-bit microcontroller (40-10kHz) is used, it results in a read time of less than 40 seconds.

**E. Kits**

In some examples, not forming part of the claimed invention, contemplated are kits comprising conformal circuits and a potentiostat. In some examples, these kits are designed to accommodate a particular target analyte, e.g., a particular protein of interest. In one example, the kit will comprise conformal circuits comprising a nanotextured porous substrate which is appropriate for the target analyte, which will have an appropriate pattern transferred to it, where the pattern is made up of an appropriate ink. In addition, the kit will further comprise a potentiostat which is calibrated to generate the data of interest to the user for the particular target analyte.

The following table illustrates examples of capture probes for which the kit may detect, the frequency of the applied electric field, the membrane type, the pore size of the membrane, and the power required:

| Type of capture probe | Frequency of electric field for Helmholtz probing | Substrate type | Pore size of membrane | Power required |
|---|---|---|---|---|
| Antibody - monoclonal | 4 Hz - 5 kHz | Track etched, acid etched, anodized, polymer, ceramic and electro deposited | 0.1 μm - 0.5 μm | 2 mW - 10 W |
| Antibody - poly clonal | 4 Hz - 5 kHz | | 0.1 μm - 0.5 μm | 2 mW - 10 W |
| Aptamer - RNA | 4 Hz - 5 kHz | | 0.05 μm - 0.2 μm | 2 mW - 10 W |
| Aptamer - protein | 7.5 Hz - 5 kHz | | 0.1 μm - 0.5 μm | 6 mW - 10 W |
| Protein | 4.8 Hz - 5 kHz | | 0.1 μm - 0.5 μm | 2 mW -10 W |
| Sugar | 4 Hz - 5 kHz | | 0.03 μm - 0.1 μm | 2 mW -10 W |
| DNA | 4 Hz - 5 kHz | | 0.05 μm - 0.3 μm | 3 mW - 9 W |
| RNA | 4 Hz - 5 kHz | | 0.05 μm - 0.3 μm | 4 mW - 10 W |
| Steroids | 4 Hz - 5 kHz | | 0.03 μm - 0.1 μm | 2 mW - 10 W |
| Cholesterol | 4 Hz - 5 kHz | | 0.03 μm - 0.1 μm | 2 mW - 10 W |

For example, a conformal circuit designed to detect C-reactive protein would have a substrate of nanoporous material, e.g., paper, having a porosity of $10^{13}$ to $10^{15}$ pores/$cm^2$ of 200 nm pores, where the circuit is made of a pattern that is interdigitated or edge-free interdigitated, or a concentric ring made using metal nanoparticle-infused carbon ink infused with gold/platinum/silver/copper/nickel. The parameters of interest that would be inputed into the potentiostat include the applied voltage of 10 mV and an applied frequency and range of 20 Hz to 10 kHz. Finally, the parameters of interest for analysis include the frequency of analysis, applied voltage, current measured, calculated impedance, estimated concentration, and standard calibration curve.

**F. Examples**

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

**EXAMPLE 1**

This assay has been used in the impedance format towards detecting Troponin-T in human serum. 0.1 pg/mL sensitivity has been achieved. Multiple replicates with data collected over a ninety day period is shown in FIG. 2. The circuit utilized a comb-based interdigitated electrical circuit, but any rectilinear combination of working and reference electrode is suitable for this application. The substrate was a nanoporous nylon membrane and the pattern was made using cryo-evaporation of gold ink, also known as gold sputtering, which is an additive deposition technique. Sample volume was 1 to 10 microliters. The circuit was connected to the impedance reader and a bias potential in the millivolt regime is applied and the change in impedance due to the step-wise introduction of the various components of the assay, linker, molecules, receptor, and ligand produces a step-wise measurable impedance change.

To calibrate the device, the inventors first deposited a thiol based linker that can effectively bind to the gold electrode. DSP dissolved in DMSO was used. A sulfur bond is formed with the gold electrode and an open amine end is left for protein/biomolecule immobilization. Following this, the inventors saturated the linker deposited sensor surface with monoclonal Troponin-T antibody. A buffer wash was performed to remove excess antibodies. Next, a blocking buffer consisting of albumin was used to close off all non-antibody immobilized linker molecules. This effectively helps in reducing noise signals due to non-specific binding of target analytes to linker sites. A buffer wash was performed to remove excess blocker molecules. The step is the baseline point or control step of the assay where zero dose of antigen is present. Antigen doses, in this case Troponin-T, were prepared in buffer media in increasing logarithmic doses. The buffer media used was phosphate buffer solution, human serum, and human plasma. Troponon-T antigen doses were spotted on the sensor surface one by one in the order of increasing concentration. Spotting refers to inoculation, pipetting out, or application of a sample on the sensor substrate. Impedance measurements were performed at each step of the assay. Impedance measurements were calculated as the ratio between applied voltage and measured current response at different frequency points in the range of 2 Hz to 15 kHz. The maximum impedance change occurred at 100.4 Hz. The impedance as a function of frequency was calculated using a Fourier transform. Applying a voltage having a frequency in which the greatest impedance change occurred, the change in impedance was calculated for every dose as the difference between impedance at baseline and at the current dose being measured. A calibration response curve was built by plotting the change in impedance and troponin-T antigen concentration. A quadratic equation was used to fit the calibration response curve. This fitting study resulted in a polynomial calibration equation that was used for estimating concentration of Troponin-T from test samples. For testing samples and estimating concentration of Troponin-T, the following assay protocol was used. The thiol based linker molecules were first deposited on the sensor surface. Monoclonal antibodies specific to Troponin-T was used for the detection. A blocking buffer was used to seal-off non-specific binding sites. Buffer washes were performed at intermediate steps to remove excess molecules not bound to the surface. The impedance measurement carried out at the buffer wash after blocking buffer application was used as the baseline or control impedance. Following this, test samples were applied to the sensor surface and impedance measurements were carried out. The test sample's impedance was calculated and was used in the quadratic equation discussed above to estimate the concentration of Troponin-T in the test samples. There was a dose dependent change to the measured impedance.

The metallic switch behavior of the conformal circuit has been mapped towards detecting trace pesticides at ultra-low concentrations. As a representative example, detection of atrazine has been demonstrated when spiked in municipal water supply. The data has been obtained from multiple replicates collected over a thirty day period (FIG. 3). The conformal circuit works like an AND gate. There are two inputs to the circuit and one output. When the input region of the circuit contains only the receptor or the ligand, then the output will remain low. However, in the presence of both the antibody and the small molecule, the output will be high, showing the turn -on of the metallic switch reaching its threshold voltage resulting in a current measurement in the micro amps range at the output. The volume used for this assay was 1 to 10 microliters.

The transfer characteristics for non-linear two terminal device behavior have been demonstrated for detecting DNA. The change in transfer charge or transconductance for various biasing voltages for the target and the associated back ground has been demonstrated (FIG. 4). The width of the conducting channel in the conformal circuit was varied and hence its current carrying capability. The width is varied due to the interaction of the targeted ligands with receptors immobilized on the semiconducting surfaces of the circuit. Surface modification is achieved using carboxylic, hydroxlic, sulfur, or amine based chemistries. Binding of the targeted species modulates the channel current. For a specific applied bias potential, the current carrying capacity of the channel is modulated by the dose dependent interactions of the targeted molecules onto the circuit. The volume used to perform this assay was 1 to 10 microliters.

**EXAMPLE 2**

Samples of miR21 enriched cells were tested on a paper cartridge, using a twenty base pair oligo-target miR21. Wild type cells were used as a control. The relative concentration of miR21 was high, i.e., greater than 200 copies/cell. These measurements were made by a nucleic acid-based sensor. The sensor was prepared by first generating the miR21 probe by in vitro transcription from a plasmid harboring a cDNA of the mature microRNA. The conformal circuit was made of a nanoporous nylon membrane patterned using cryo-evaporation with gold ink. The nucleic acid probe, complementary to a region of miR21, was bound to the gold electrode. In the electrical oligonucleotide assay protocol, this configuration permitted capture, detection, and quantification of miR21 in RNA isolates from cell lysates.

**EXAMPLE 3**

**Septicemia:** Samples taken from patients to diagnosis the pathogenic basis of septicemia. Markers were used for lipopolysaccaride (indicator of gram negative bacteria), lipoteichoic acid (indicator of gram positive bacteria), and procalcitonin (marker of severe sepsis caused by bacteria and generally grades well with the degree of sepsis). Samples included five whole blood samples from ICU patients who had a clinical confirmation of septicemia.

To calibrate the device, the inventors first deposited a thiol based linker that can effectively bind to the gold electrode. DSP dissolved in DMSO was used. A sulfur bond is formed with the gold electrode and an open amine end is left for protein/biomolecule immobilization. Following this, the inventors saturated the linker deposited sensor surface with monoclonal antibodies for lipopolysaccharide, lipoteichoic acid, and procalcitonin. A buffer wash was performed to remove excess antibodies. Next, a blocking buffer consisting of albumin was used to close off all non-antibody immobilized linker molecules. This effectively helps in reducing noise signals due to non-specific binding of target analytes to linker sites. A buffer wash was performed to remove excess blocker molecules. This is the baseline point or control step of the assay where zero dose of antigen is present. Antigen/protein biomarker doses were prepared in buffer media in increasing logarithmic doses. The buffer media used was phosphate buffer solution, human serum, and human plasma. Each of these buffer media were applied to a separate sensor. Antigen/protein biomarker doses were spotted on the sensor surface one by one in the order of increasing concentration. Impedance measurements were performed at each step of the assay. Impedance measurements were calculated as the ratio between applied voltage and measured current response at different frequency points in the range of 2 Hz to 15 kHz. Lipopolysaccharides showed the greatest impedance change at 99.3 Hz, lipoteichoic acid showed the greatest impedance change at 120 Hz, and procalcitonin showed the greatest impedance change at 110 Hz. The impedance as a function of frequency was calculated using a Fourier transform. Applying a voltage having a frequency in which the greatest impedance change occurred, the change in impedance was calculated for every dose as the difference between impedance at baseline and at the current dose being measured. A calibration response curve was built by plotting the change in impedance and antigen concentration. A quadratic equation was used to fit the calibration response curve. This fitting study resulted in a polynomial calibration equation that was used for estimating concentration of antigen/protein biomarkers from test samples. For testing samples and estimating concentration of the protein biomarkers, the following assay protocol was used. The thiol based linker molecules were first deposited on the sensor surface. Monoclonal antibodies specific to the protein biomarkers were used for the detection. A blocking buffer was used to seal-off non-specific binding sites. Buffer washes were performed at intermediate steps to remove excess molecules not bound to the surface. The impedance measurement carried out at the buffer wash after blocking buffer application was used as the baseline or control impedance. Following this, test samples were applied to the sensor surface and impedance measurements were carried out. The test sample's impedance was calculated and was used in the quadratic equation discussed above to estimate the concentration of the protein biomarkers in the test samples.

Quantitative results were obtained in less than twenty minutes, with the lowest detection limit for the markers at 10 fg/mL. See Table 1. A correlation of severe septicemia to high levels of procalcitonin was identified.

**Table 1**

| Sample | Procalcitonin (pg/mL) | Lipopolysaccharide - pg/mL (gram negative bacteria) | Lipoteichoic acid - pg/mL (gram positive bacteria) | Clinical data |
|---|---|---|---|---|
| 1 | 0.026 | 0.08 | < 0.01 | Yes |
| 2 | 43.97 | 0.93 | 12.6 | Yes |
| 3 | 126.83 | 18.64 | 184.91 | Yes |
| 4 | 635.99 | 194.60 | 340.44 | Yes, severe |
| 5 | 1794 | 991.00 | 3716 | Yes, severe |

The conformal circuit was made of a nanoporous nylon membrane patterned using cryo-evaporation with gold ink. The protocol used was an electrical immunoassay protocol which involves binding a protein specific monoclonal antibody to the substrate electrodes. Blood samples from patients who were suspected with septic infection were collected. These were tested for three different markers Procalcitonin, Lipopolysaccharide and Lipoteichoic acid. The detection was performed by studying impedance changes as a result of specific protein markers binding to the immobilized capture antibodies on the surface of the electrodes.

**Cardiovascular markers:** Twelve human plasma samples from patients who have had myocardial infarction events were tested to quantify troponin-T (cardiac marker) for analyzing its behavior and reliability as a marker for early diagnosis.

To calibrate the device, the inventors first deposited a thiol based linker that can effectively bind to the gold electrode. DSP dissolved in DMSO was used. A sulfur bond is formed with the gold electrode and an open amine end is left for protein/biomolecule immobilization. Following this, the inventors saturated the linker deposited sensor surface with monoclonal Troponin-T antibody. A buffer wash was performed to remove excess antibodies. Next, a blocking buffer consisting of albumin was used to close off all non-antibody immobilized linker molecules. This effectively helps in reducing noise signals due to non-specific binding of target analytes to linker sites. A buffer wash was performed to remove excess blocker molecules. The step is the baseline point or control step of the assay where zero dose of antigen is present. Antigen doses, in this case Troponin-T, was prepared in buffer media in increasing logarithmic doses. The buffer media used was phosphate buffer solution, human serum and human plasma. Troponin-T antigen doses were spotted on the sensor surface one by one in the order of increasing concentration. Impedance measurements were performed at each step of the assay. Impedance measurements were calculated as the ratio between applied voltage and measured current response at different frequency points in the range of 2 Hz to 15 kHz. The impedance as a function of frequency was calculated using a Fourier transform. Applying a voltage having a frequency in which the greatest impedance change occurred, the change in impedance was calculated for every dose as the difference between impedance at baseline and at the current dose being measured. A calibration response curve was built by plotting the change in impedance and troponin-T antigen concentration. A quadratic equation was used to fit the calibration response curve. This fitting study resulted in a polynomial calibration equation that was used for estimating concentration of Troponin-T from test samples. For testing samples and estimating concentration of Troponin-T, the following assay protocol was used. The thiol based linker molecules were first deposited on the sensor surface. Monoclonal antibodies specific to Troponin-T was used for the detection. A blocking buffer was used to seal-off non-specific binding sites. Buffer washes were performed at intermediate steps to remove excess molecules not bound to the surface. The impedance measurement carried out at the buffer wash after blocking buffer application was used as the baseline or control impedance. Following this, test samples were applied to the sensor surface and impedance measurements were carried out. The test sample's impedance was calculated and was used in the quadratic equation discussed above to estimate the concentration of the protein biomarkers in the test samples.

The lowest detected dose was 0.71 pg/mL, which is three orders of magnitude more sensitive than ELISA for this marker. FIG. 6. The conformal circuit was made of a nanoporous nylon membrane patterned using cryo-evaporation with gold ink. The protocol used was an electrical immunoassay protocol which involves binding a protein specific monoclonal antibody to the substrate electrodes. In this case, it was antibody to Troponin-T protein biomarker. Plasma samples from twelve patients were collected. These samples were applied to the sensing substrate. The presence and amount of troponin-T were quantified by measuring the impedance response as a result of Troponin-T binding to the antibodies immobilized on the electrode surface.

**Cancer markers:** Ten human serum matrix samples spiked with prostate-specific antigen (PSA) were tested to quantify PSA for its use in diagnosis of prostate cancer.

To calibrate the device, the inventors first deposited a thiol based linker that can effectively bind to the gold electrode. DSP dissolved in DMSO was used. A sulfur bond is formed with the gold electrode and an open amine end is left for protein/biomolecule immobilization. Following this, the inventors saturated the linker deposited sensor surface with monoclonal prostate specific antigen antibody. A buffer wash was performed to remove excess antibodies. Next, a blocking buffer consisting of bovine serum albumin with proprietary thermoscientific reagents was used to close off all non-antibody immobilized linker molecules. This effectively helps in reducing noise signals due to non-specific binding of target analytes to linker sites. A buffer wash was performed to remove excess blocker molecules. The step is the baseline point or control step of the assay where zero dose of antigen is present. Antigen doses, in this case, prostate specific antigen was prepared in buffer media in increasing logarithmic doses. The buffer media used was phosphate buffer solution, human serum matrix and human plasma. Prostate specific antigen doses were spotted on the sensor surface one by one in the order of increasing concentration. Impedance measurements were performed at each step of the assay. Impedance measurements were calculated as the ratio between applied voltage and measured current response at different frequency points in the range of 2 Hz to 15 kHz. The impedance as a function of frequency was calculated using a Fourier transform. PSA showed the greatest impedance change at 128.4 Hz. Applying a voltage having a frequency in which the greatest impedance change occurred, the change in impedance was calculated for every dose as the difference between impedance at baseline and at the current dose being measured. A calibration response curve was built by plotting the change impedance and prostate specific antigen concentration. A quadratic equation was used to fit the calibration response curve. This fitting study resulted in a polynomial calibration equation that was used for estimating concentration of prostate specific antigen from test samples. For testing samples and estimating concentration of prostate specific antigen, the following assay protocol was used. The thiol based linker molecules were first deposited on the sensor surface. Monoclonal antibodies specific to prostate specific antigen was used for the detection. A blocking buffer was used to seal-off non-specific binding sites. Buffer washes were performed at intermediate steps to remove excess molecules not bound to the surface. The impedance measurement carried out at the buffer wash after blocking buffer application was used as the baseline or control impedance. Following this, test samples were applied to the sensor surface and impedance measurements were carried out. The test sample's impedance was calculated and was used in the quadratic equation discussed above to estimate the concentration of the protein biomarkers in the test samples.

The lowest detected dose was 0.0052 ng/mL. FIG. 7. The conformal circuit was made of a nanoporous nylon membrane patterned using cryo-evaporation with gold ink. The protocol used was an electrical immunoassay protocol which involves binding a protein specific monoclonal antibody to the substrate electrodes. In this case, it was antibody to prostate specific antigen biomarker. Human serum matrix samples were prepared and spiked with different concentrations of prostate specific antigen. These samples were applied to the sensing substrate. The presence and amount of Prostate specific antigen were quantified by measuring the impedance response as a result of prostate specific antigen binding to the antibodies immobilized on the electrode surface.

**Fungicide detection:** 6 juice samples spiked with strobulrin fungicides were tested to quantify the level of strobulrin in the sample. The lowest detected dose was 10 pM. Table 2.

**Table 2**

| Sample | Spectrophotometry (Trifloxystrobin) | impress (Trifloxystrobin) | Spectrophotometry (Azoxystrobin) | impress (Azoxystrobin) |
|---|---|---|---|---|
| 1 | Cannot be detected | 10 pM | Cannot be detected | 13 pM |
| 2 | 101 pM | 100 pM | 125 pM | 126.2 pM |
| 3 | 150 pM | 150 pM | 142 oM | 144 pM |
| 4 | 10 nM | 10 nM | 19 nM | 19.2 nM |
| 5 | 100 nM | 100 nM | 100 nM | 100 nM |
| 6 | 1000 nM | 1000 nM | 800 nM | 800 nM |

The conformal circuit was made of a nanoporous nylon membrane patterned using cryo-evaporation with gold ink. The protocol used was an electrical immunoassay protocol which involves binding a fungicide specific antibody or aptamer to the sensing substrate. Fresh juice samples spiked with various concentrations of the mentioned fungicides were taken and applied to the sensor substrate.

To calibrate the device, the inventors first deposited a thiol based linker that can effectively bind to the gold electrode. DSP dissolved in DMSO was used. A sulfur bond is formed with the gold electrode and an open amine end is left for protein/biomolecule immobilization. Following this, the inventors saturated the linker deposited sensor surface with monoclonal antibodies or aptamers. A buffer wash was performed to remove excess antibodies. Next, a blocking buffer consisting of albumin was used to close off all non-antibody immobilized linker molecules. This effectively helps in reducing noise signals due to non-specific binding of target analytes to linker sites. A buffer wash was performed to remove excess blocker molecules. The step is the baseline point or control step of the assay where zero dose of antigen is present. Fungicide doses were prepared in buffer media in increasing logarithmic doses. The buffer media used was phosphate buffer solution, water or juice varieties. Fungicide doses were spotted on the sensor surface one by one in the order of increasing concentration. Impedance measurements were performed at each step of the assay. Impedance measurements were calculated as the ratio between applied voltage and measured current response at different frequency points in the range of 2 Hz to 15 kHz. The impedance as a function of frequency was calculated using a Fourier transform. Fungicide showed the greatest impedance change at 104 Hz. Applying a voltage having a frequency in which the greatest impedance change occurred, the change in impedance was calculated for every dose as the difference between impedance at baseline and at the current dose being measured. A calibration response curve was built by plotting change in impedance and fungicide concentration. A quadratic equation was used to fit the calibration response curve. This fitting study resulted in a polynomial calibration equation that was used for estimating concentration of fungicide from test samples. For testing samples and estimating concentration of fungicide, the following assay protocol was used. The thiol based linker molecules were first deposited on the sensor surface. Monoclonal antibodies specific to fungicides was used for the detection. A blocking buffer was used to seal-off non-specific binding sites. Buffer washes were performed at intermediate steps to remove excess molecules not bound to the surface. The impedance measurement carried out at the buffer wash after blocking buffer application was used as the baseline or control impedance. Following this, test samples were applied to the sensor surface and impedance measurements were carried out. The test sample's impedance was calculated and was used in the quadratic equation discussed above to estimate the concentration of the protein biomarkers in the test samples.

The presence of the various fungicides was detected and quantified by measuring the impedance changes as a result of fungicide binding to the aptamers or antibodies immobilized on the electrode surface. Aptamers, or oligonucleotide probes, can used for capture and detection of biomarkers and biomolecules.

## REFERENCES

The following references provide exemplary procedural or other details supplementary to those set forth herein.

Reighard & Barendt, "Conformal Coating Process Controls: The Manufacturing Engineer's Aid." APEX. Long Beach, CA. March 2000.

Vestergaard, et al., Sensors. 7(12):3442-58, 2007.

## Claims

1. A method of detecting or quantifying a target analyte in a sample using a handheld measuring device and a conformal analyte sensor circuit comprising the steps of:

    (a) providing a conformal analyte sensor circuit comprising:

        a solid substrate having a top surface comprising a porous nanotextured substrate; and
        a conductive material conformally patterned on the top surface of the solid substrate in a circuit design, thereby creating a conformal circuit comprising electrodes, wherein the electrodes consist of a reference electrode (108) and a working electrode (106) and wherein the conformal analyte sensor circuit further comprises a redox material bound with a receptor of the target analyte immobilized onto the conformal circuit;

    (b) applying an alternating input electric voltage between the reference electrode (108) and the working electrode (106) of the conformal analyte sensor circuit;
    (c) varying a frequency of the alternating input electric voltage between a minimum frequency and a maximum frequency;
    (d) amplifying an output current flowing between the reference electrode (108) and the working electrode (106) using a programmable gain amplifier (102);
    (e) calculating an impedance by comparing the input electric voltage to the output current using a programmable microcontroller (100); and
    (f) detecting a target analyte or calculating a target analyte concentration from the calculated impedance using a programmable microcontroller (100).

2. The method of claim 1, wherein the input electric voltage has a minimum frequency of 2 or 50 Hz and a maximum frequency of 15 kHz, and optionally wherein the frequency of the input electric voltage is varied in 2 Hz increments.

3. The method of claim 1 wherein the input electric voltage is sinusoidal, a sawtooth wave, a square wave, or a triangle wave.

4. The method of claim 1, wherein the input electric voltage is between 1 mV and 10 V, between 1 mV and 100 mV, or between 100 mV and 10 V.

5. The method of claim 1, wherein the output current is between 10 pA and 10 mA, between 10 pA and 100 nA, or between 100 nA and 10 mA.

6. The method of claim 1, wherein the output current is amplified by a factor between 1 and 200.

7. The method of claim 1, further comprising calculating a difference in phase between the input electric voltage and the output current using a programmable microcontroller (100).

8. The method of claim 1, further comprising calculating impedance as a function of frequency by applying a Fourier transform using a programmable microcontroller (100).

9. The method of claim 1, further comprising calculating impedance using Lissajous curves using a programmable microcontroller (100), or calculating impedance as a function of frequency using multi-slice splitting and signal analysis.

10. The method of claim 1, further comprising displaying the calculated target analyte concentration on an LCD display (104).

11. The method of claim 1, further comprising displaying an output on an LCD display (104) or on a smartphone (300).

12. The method of claim 1, wherein the calculated impedance is non-faradaic.

**Patentansprüche**

1. Verfahren zum Nachweisen oder zum Quantifizieren eines Zielanalyten in einer Probe unter Verwendung einer tragbaren Messvorrichtung und einer konformen Analytsensorschaltung, umfassend die Schritte:

   (a) Bereitstellen einer konformen Analytsensorschaltung, umfassend:

      ein festes Substrat, das eine obere Oberfläche aufweist, umfassend ein poröses nanotexturiertes Substrat; und
      ein leitendes Material, das auf der oberen Oberfläche des festen Substrats in einem Schaltungsaufbau konform gemustert ist, wobei dadurch eine konforme Schaltung erzeugt wird, umfassend die Elektroden, wobei die Elektroden aus einer Referenzelektrode (108) und einer Arbeitselektrode (106) bestehen und wobei die konforme Analytsensorschaltung ferner ein Redoxmaterial umfasst, das an einen Rezeptor des Zielanalyten gebunden ist, der auf der konformen Schaltung immobilisiert ist;

   (b) Anwenden einer elektrischen Wechseleingangsspannung zwischen der Referenzelektrode (108) und der Arbeitselektrode (106) der konformen Analytsensorschaltung;
   (c) Variieren einer Frequenz der elektrischen Wechseleingangsspannung zwischen einer minimalen Frequenz und einer maximalen Frequenz;
   (d) Verstärken eines Ausgangsstroms, der zwischen der Referenzelektrode (108) und der Arbeitselektrode (106) fließt, unter Verwendung eines programmierbaren Verstärkers (102);
   (e) Berechnen einer Impedanz durch Vergleichen der elektrischen Eingangsspannung mit dem Ausgangsstrom unter Verwendung eines programmierbaren Mikrocontrollers (100); und
   (f) Nachweisen eines Zielanalyten oder Berechnen einer Zielanalytkonzentration aus der berechneten Impedanz unter Verwendung eines programmierbaren Mikrocontrollers (100).

2. Verfahren nach Anspruch 1, wobei die elektrische Eingangsspannung eine minimale Frequenz von 2 oder 50 Hz und eine maximale Frequenz von 15 kHz aufweist und optional wobei die Frequenz der elektrischen Eingangsspannung in Abständen von 2 Hz variiert wird.

3. Verfahren nach Anspruch 1, wobei die elektrische Eingangsspannung sinusförmig, eine Sägezahnwelle, eine Rechteckwelle oder eine Dreieckwelle ist.

4. Verfahren nach Anspruch 1, wobei die elektrische Eingangsspannung zwischen 1 mV und 10 V, zwischen 1 mV und 100 mV oder zwischen 100 mV und 10 V liegt.

5. Verfahren nach Anspruch 1, wobei der Ausgangsstrom zwischen 10 pA und 10 mA, zwischen 10 pA und 100 nA oder zwischen 100 nA und 10 mA liegt.

6. Verfahren nach Anspruch 1, wobei der Ausgangsstrom um einen Faktor zwischen 1 und 200 verstärkt wird.

7. Verfahren nach Anspruch 1, ferner umfassend das Berechnen einer Phasenverschiebung zwischen der elektrischen Eingangsspannung und dem Ausgangsstrom unter Verwendung eines programmierbaren Mikrocontrollers (100).

8. Verfahren nach Anspruch 1, ferner umfassend das Berechnen der Impedanz in Abhängigkeit von einer Frequenz durch Anwenden einer Fourier-Transformation unter Verwendung eines programmierbaren Mikrocontrollers (100).

9. Verfahren nach Anspruch 1, ferner umfassend das Berechnen der Impedanz unter Verwendung von Lissajous-Kurven unter Verwendung eines programmierbaren Mikrocontrollers (100) oder das Berechnen der Impedanz in Abhängigkeit von der Frequenz unter Verwendung von Mehrschichtaufspaltung und Signalanalyse.

10. Verfahren nach Anspruch 1, ferner umfassend ein Anzeigen der berechneten Zielanalytkonzentration auf einer LCD-Anzeige (104).

11. Verfahren nach Anspruch 1, ferner umfassend das Anzeigen einer Ausgabe auf einer LCD-Anzeige (104) oder auf einem Smartphone (300).

12. Verfahren nach Anspruch 1, wobei die berechnete Impedanz nicht-faradisch ist.

## Revendications

1. Procédé de détection ou de quantification d'un analyte cible dans un échantillon à l'aide d'un dispositif de mesure portatif et d'un circuit de capteur d'analyte conforme comprenant les étapes consistant à :

(a) fournir un circuit de capteur d'analyte conforme comprenant :

un substrat solide ayant une surface supérieure comprenant un substrat nanotexturé poreux ; et
un matériau conducteur modelé de manière conforme sur la surface supérieure du substrat solide dans un modèle de circuit, créant ainsi un circuit conforme comprenant des électrodes, dans lequel les électrodes sont constituées d'une électrode de référence (108) et d'une électrode de travail (106) et dans lequel le circuit de capteur d'analyte conforme comprend en outre un matériau redox lié à un récepteur de l'analyte cible immobilisé sur le circuit conforme ;

(b) appliquer une tension électrique d'entrée alternative entre l'électrode de référence (108) et l'électrode de travail (106) du circuit de capteur d'analyte conforme ;
(c) faire varier une fréquence de la tension électrique d'entrée alternative entre une fréquence minimale et une fréquence maximale ;
(d) amplifier un courant de sortie circulant entre l'électrode de référence (108) et l'électrode de travail (106) à l'aide d'un amplificateur à gain programmable (102) ;
(e) calculer une impédance en comparant la tension électrique d'entrée au courant de sortie à l'aide d'un micro-dispositif de commande programmable (100) ; et
(f) détecter un analyte cible ou calculer une concentration d'analyte cible à partir de l'impédance calculée à l'aide d'un micro-dispositif de commande programmable (100).

2. Procédé selon la revendication 1, dans lequel la tension électrique d'entrée a une fréquence minimale de 2 ou 50 Hz et une fréquence maximale de 15 kHz, et éventuellement dans lequel la fréquence de la tension électrique d'entrée varie par incréments de 2 Hz.

3. Procédé selon la revendication 1, dans lequel la tension électrique d'entrée est sinusoïdale, une onde en dents de scie, une onde carrée ou une onde triangulaire.

4. Procédé selon la revendication 1, dans lequel la tension électrique d'entrée est comprise entre 1 mV et 10 V, entre 1 mV et 100 mV, ou entre 100 mV et 10 V.

5. Procédé selon la revendication 1, dans lequel le courant de sortie est compris entre 10 pA et 10 mA, entre 10 pA et 100 nA, ou entre 100 nA et 10 mA.

6. Procédé selon la revendication 1, dans lequel le courant de sortie est amplifié d'un facteur entre 1 et 200.

7. Procédé selon la revendication 1, comprenant en outre le calcul d'une différence de phase entre la tension électrique d'entrée et le courant de sortie à l'aide d'un micro-dispositif de commande programmable (100).

8. Procédé selon la revendication 1, comprenant en outre le calcul de l'impédance en tant que fonction de la fréquence en appliquant une transformée de Fourier à l'aide d'un micro-dispositif de commande programmable (100).

9. Procédé selon la revendication 1, comprenant en outre le calcul de l'impédance à l'aide de courbes de Lissajous à l'aide d'un micro-dispositif de commande programmable (100), ou le calcul de l'impédance en tant que fonction de la fréquence à l'aide de la division multicoupe et de l'analyse de signal.

10. Procédé selon la revendication 1, comprenant en outre l'affichage de la concentration d'analyte cible calculée sur un affichage LCD (104).

11. Procédé selon la revendication 1, comprenant en outre l'affichage d'une sortie sur un affichage LCD (104) ou sur un smartphone (300).

12. Procédé selon la revendication 1, dans lequel l'impédance calculée est non-faradique.

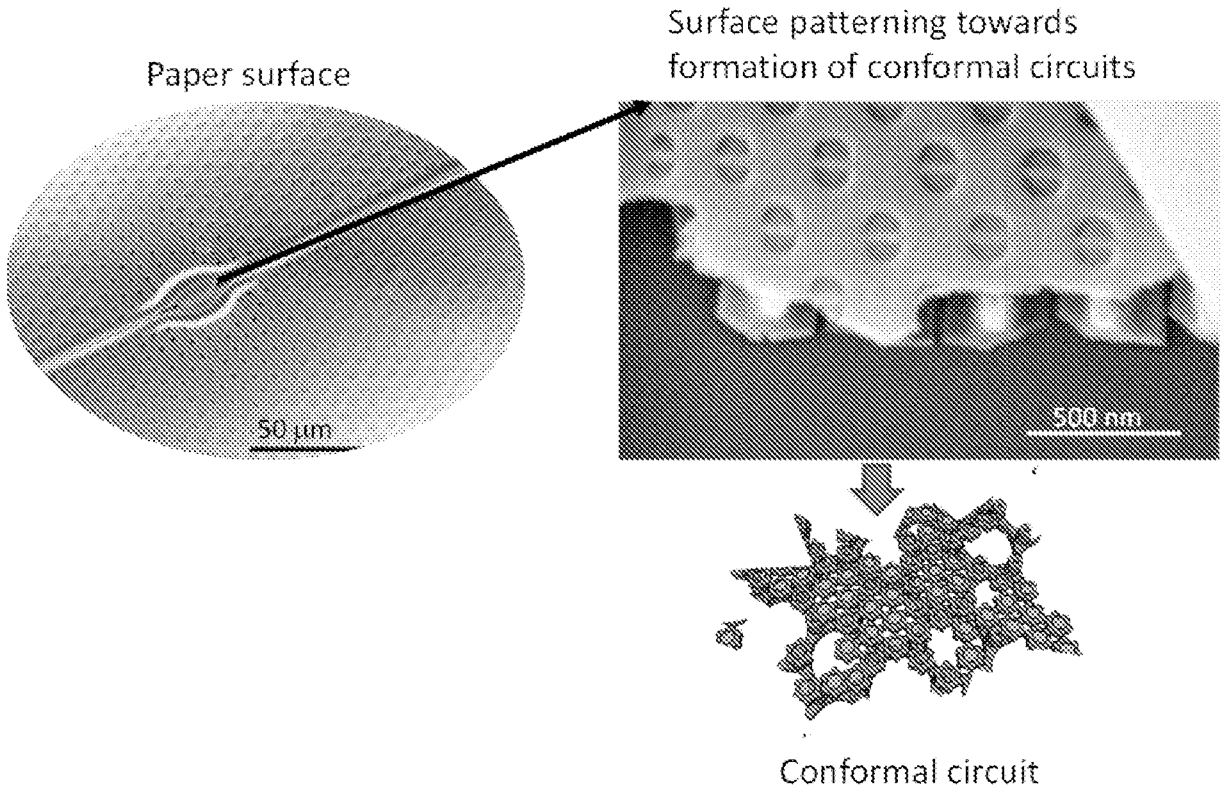
Paper surface
Surface patterning towards formation of conformal circuits
50 µm
500 nm
Conformal circuit

FIG. 1

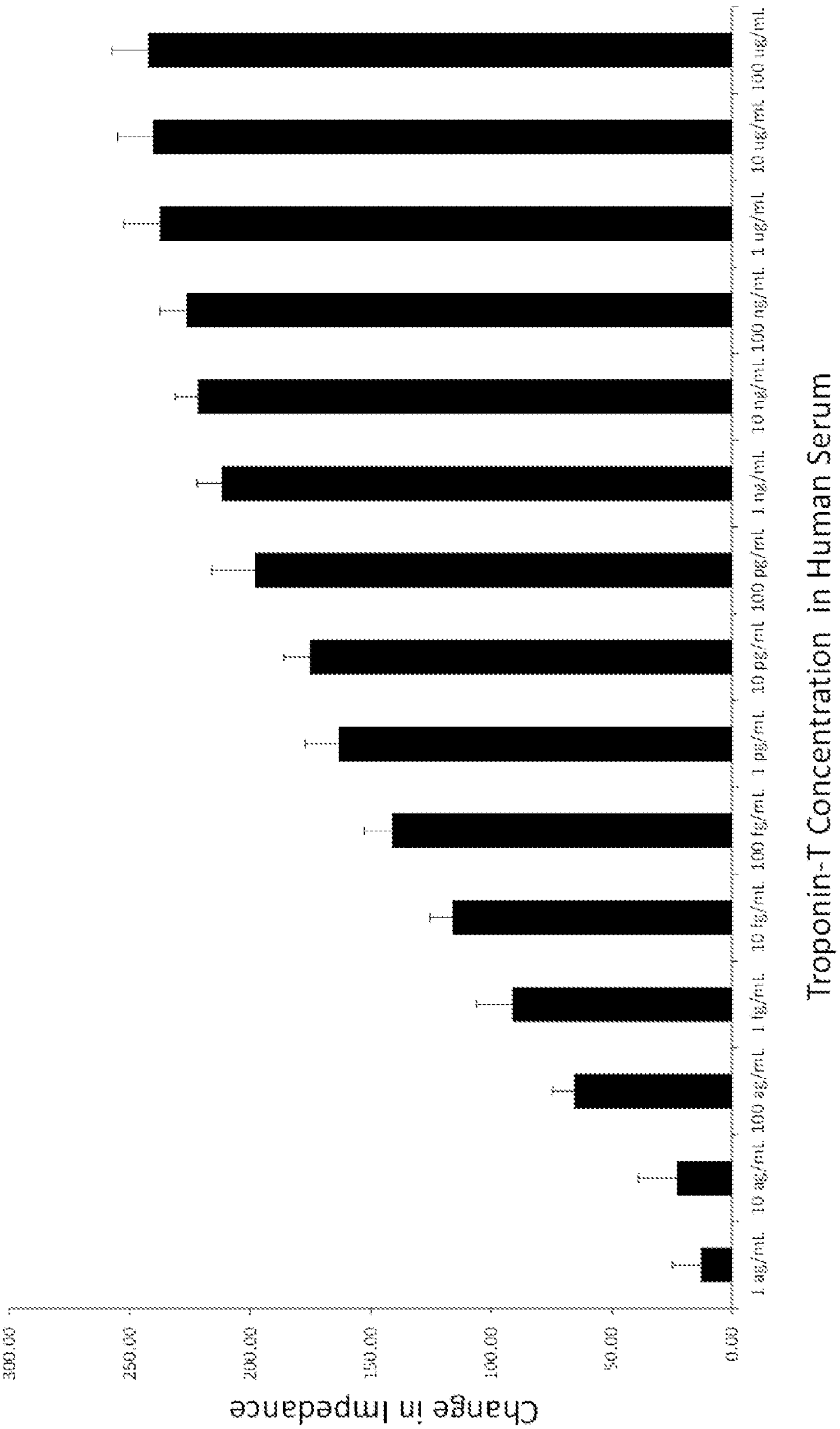
Change in Impedance
0.00
50.00
100.00
150.00
200.00
250.00
300.00
1 ag/mL
10 ag/mL
100 ag/mL
1 fg/mL
10 fg/mL
100 fg/mL
1 pg/mL
10 pg/mL
100 pg/mL
1 ng/mL
10 ng/mL
100 ng/mL
1 ug/mL
10 ug/mL
100 ug/mL
Troponin-T Concentration in Human Serum

FIG. 2

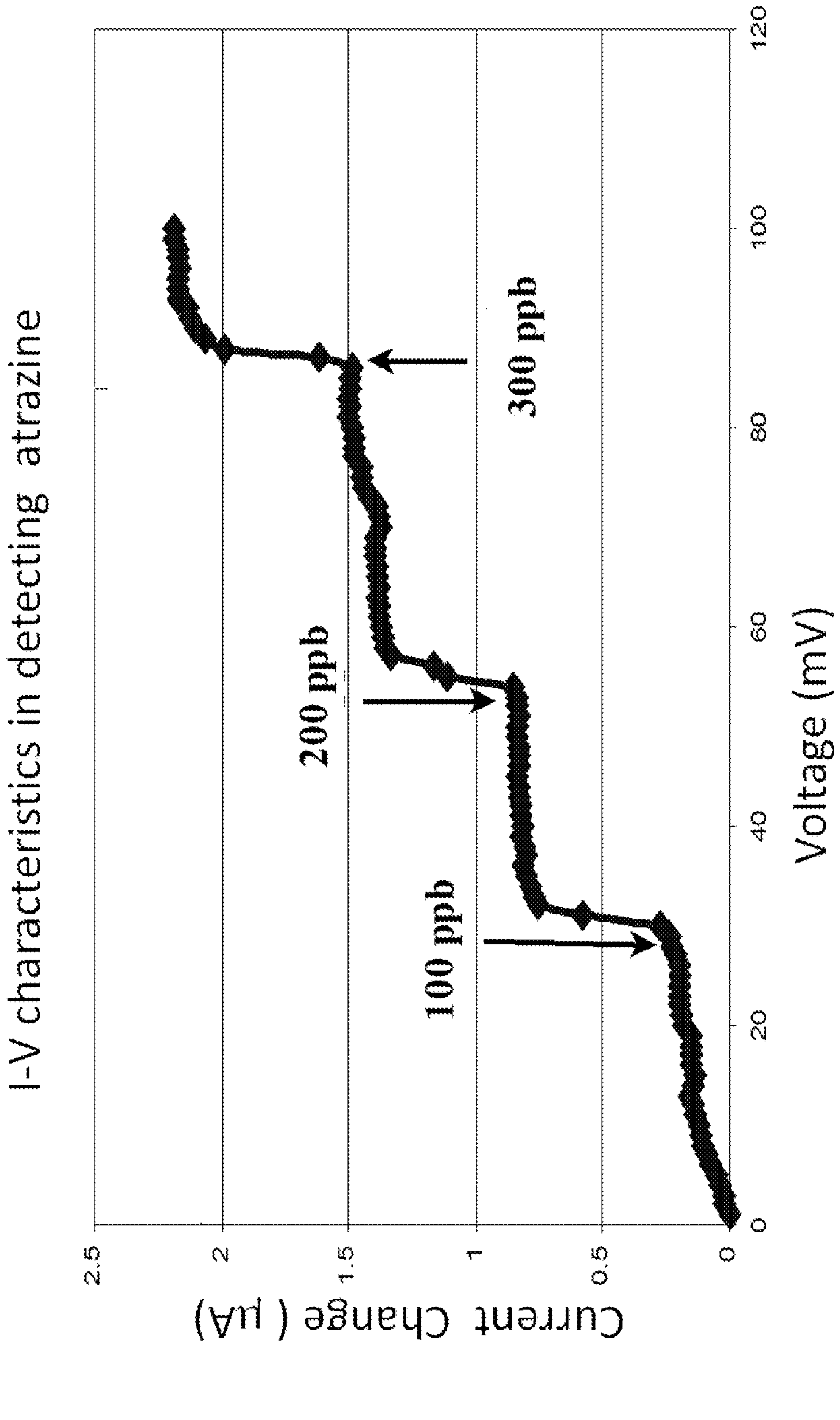

I-V characteristics in detecting atrazine
Current Change ( μA)
Voltage (mV)
0
0.5
1
1.5
2
2.5
0
20
40
60
80
100
120
100 ppb
200 ppb
300 ppb


FIG. 3

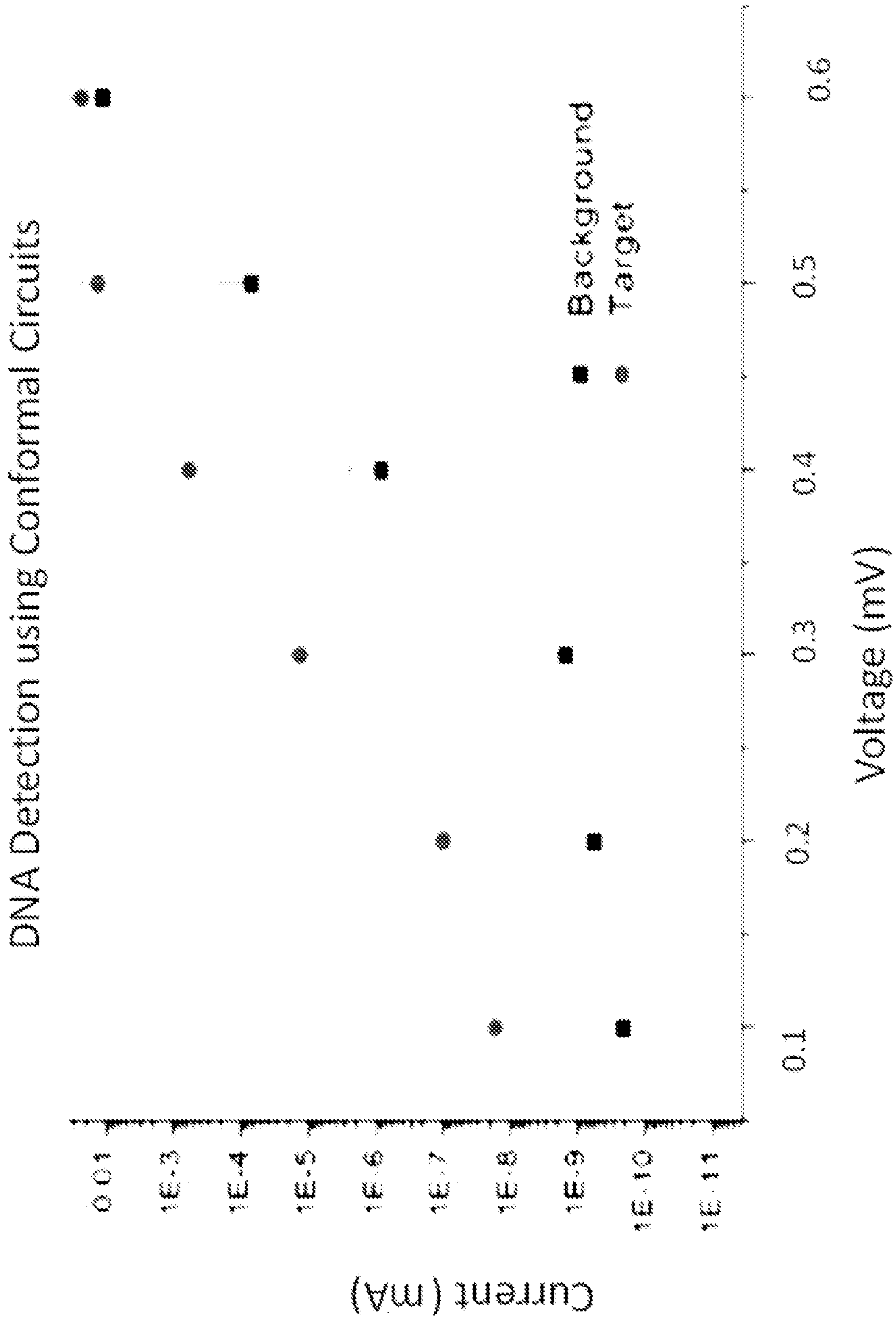
DNA Detection using Conformal Circuits
Background
Target
Current ( mA)
0.01
1E-3
1E-4
1E-5
1E-6
1E-7
1E-8
1E-9
1E-10
1E-11
Voltage (mV)
0.1
0.2
0.3
0.4
0.5
0.6

FIG. 4

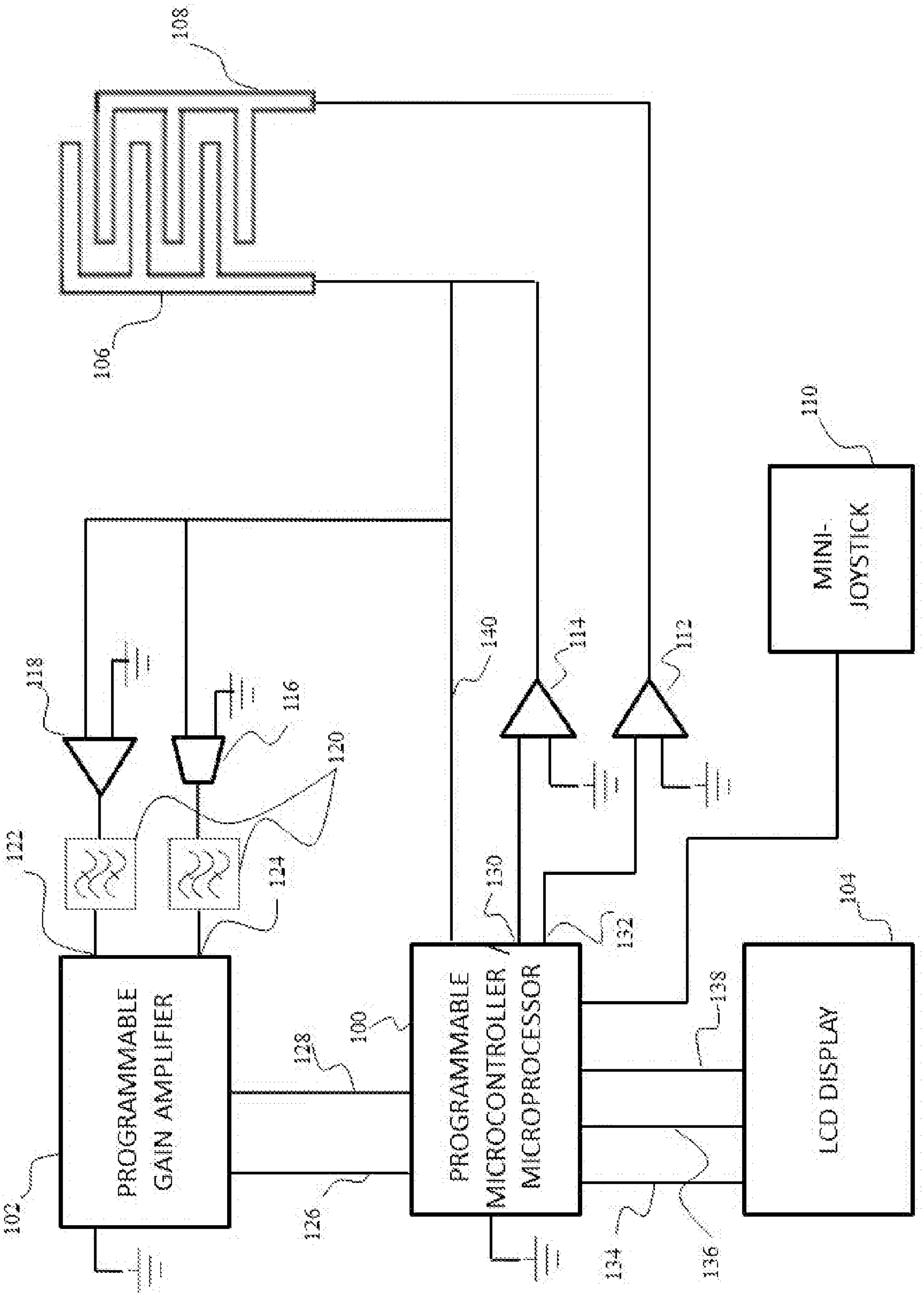
PROGRAMMABLE GAIN AMPLIFIER
PROGRAMMABLE MICROCONTROLLER MICROPROCESSOR
LCD DISPLAY
MINI-JOYSTICK
100
102
104
106
108
110
112
114
116
118
120
122
124
126
128
130
132
134
136
138
140

**FIG. 5**

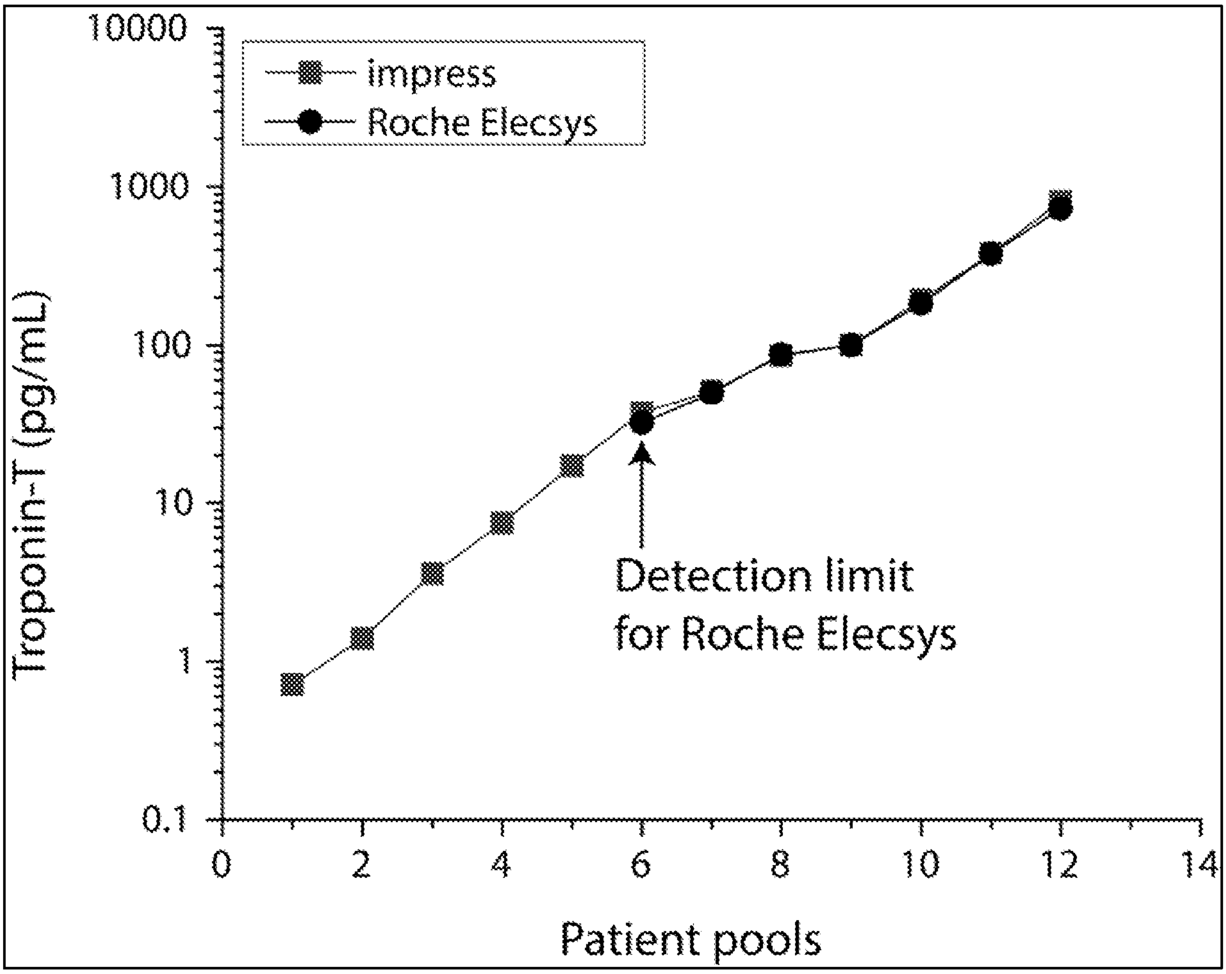
10000
1000
100
10
1
0.1
Troponin-T (pg/mL)
impress
Roche Elecsys
Detection limit
for Roche Elecsys
0
2
4
6
8
10
12
14
Patient pools

FIG. 6

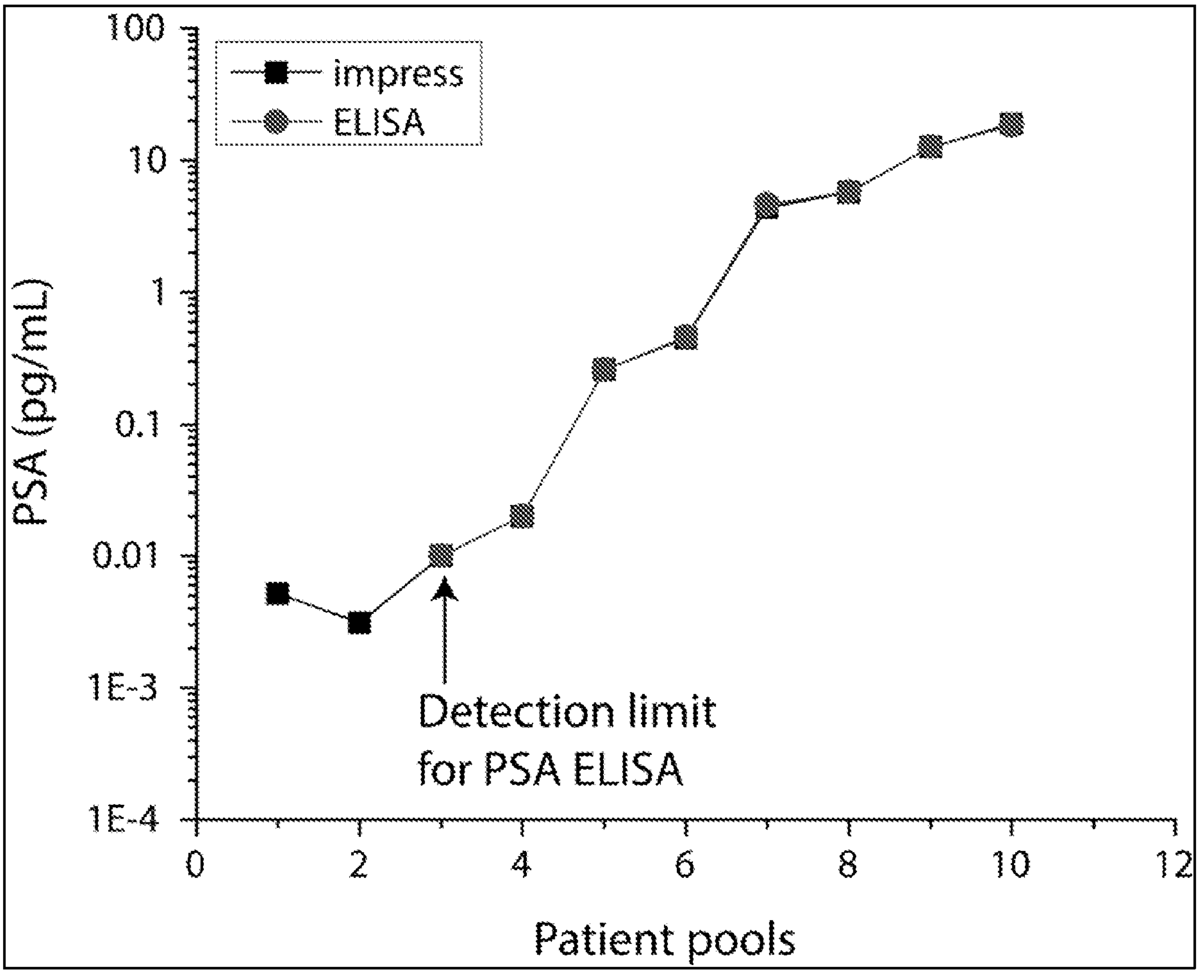
100
10
1
0.1
0.01
1E-3
1E-4
PSA (pg/mL)
impress
ELISA
Detection limit
for PSA ELISA
0
2
4
6
8
10
12
Patient pools

FIG. 7

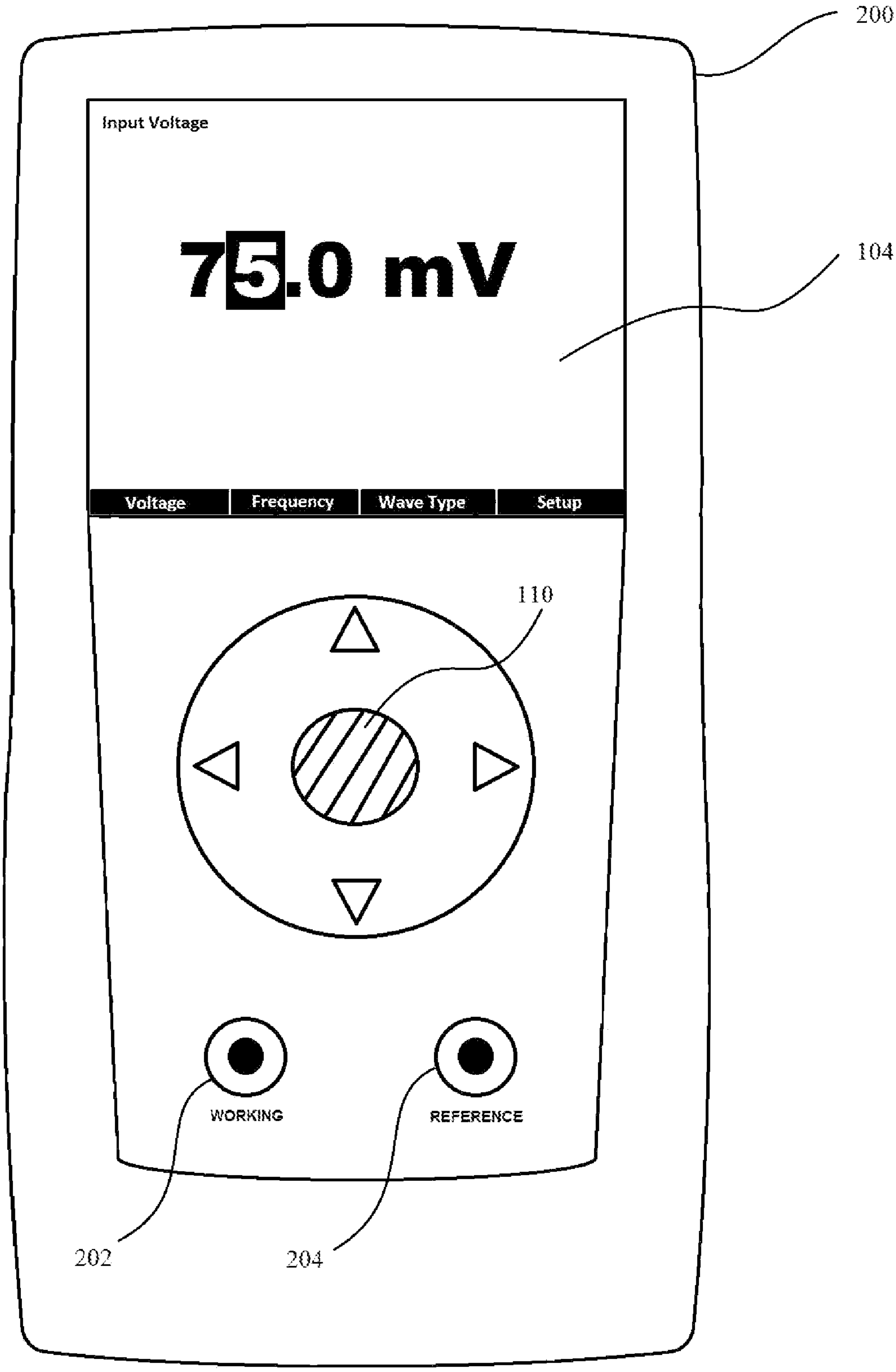
200
Input Voltage
75.0 mV
104
Voltage
Frequency
Wave Type
Setup
110
WORKING
REFERENCE
202
204

FIG. 8

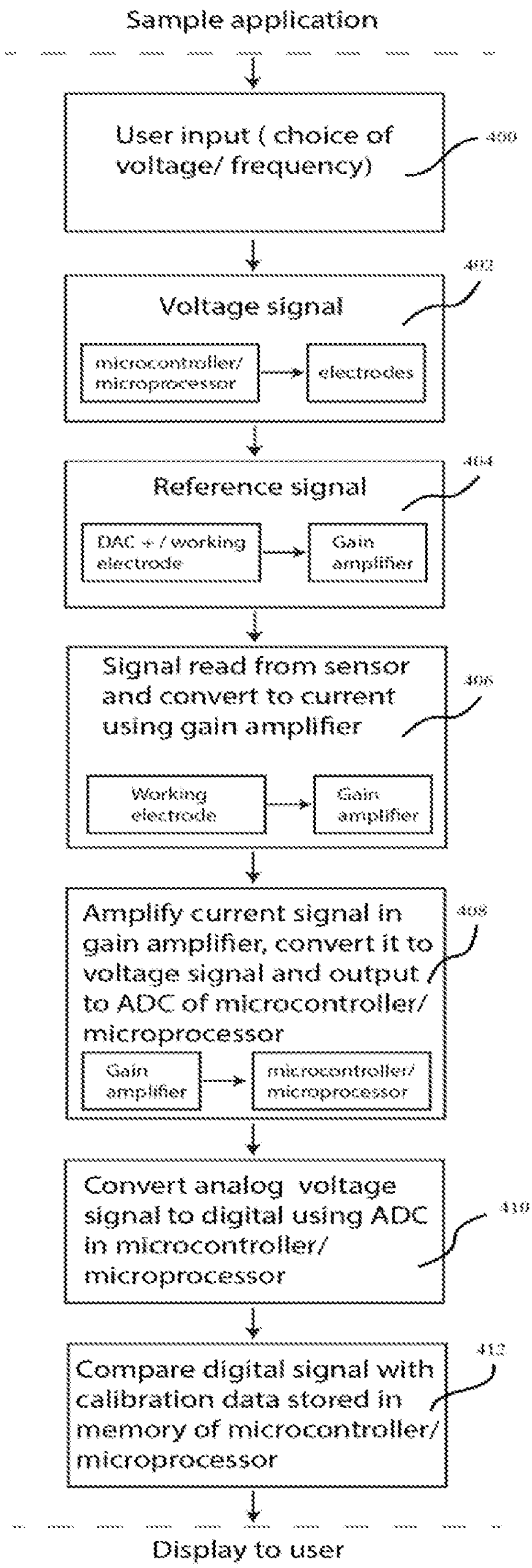
Sample application
User input ( choice of voltage/ frequency)
400
Voltage signal
402
microcontroller/ microprocessor
electrodes
Reference signal
404
DAC + / working electrode
Gain amplifier
Signal read from sensor and convert to current using gain amplifier
406
Working electrode
Gain amplifier
Amplify current signal in gain amplifier, convert it to voltage signal and output to ADC of microcontroller/ microprocessor
408
Gain amplifier
microcontroller/ microprocessor
Convert analog voltage signal to digital using ADC in microcontroller/ microprocessor
410
Compare digital signal with calibration data stored in memory of microcontroller/ microprocessor
412
Display to user

FIG. 9

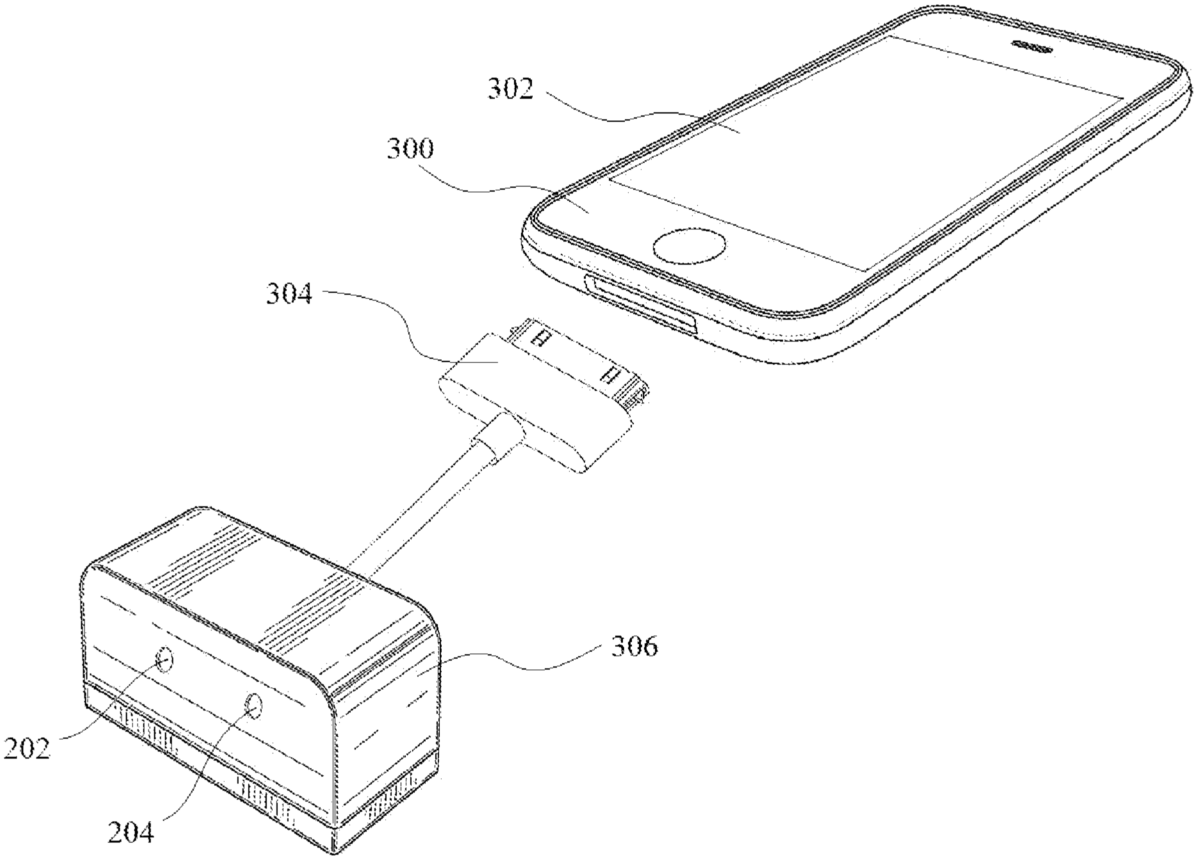


FIG. 10

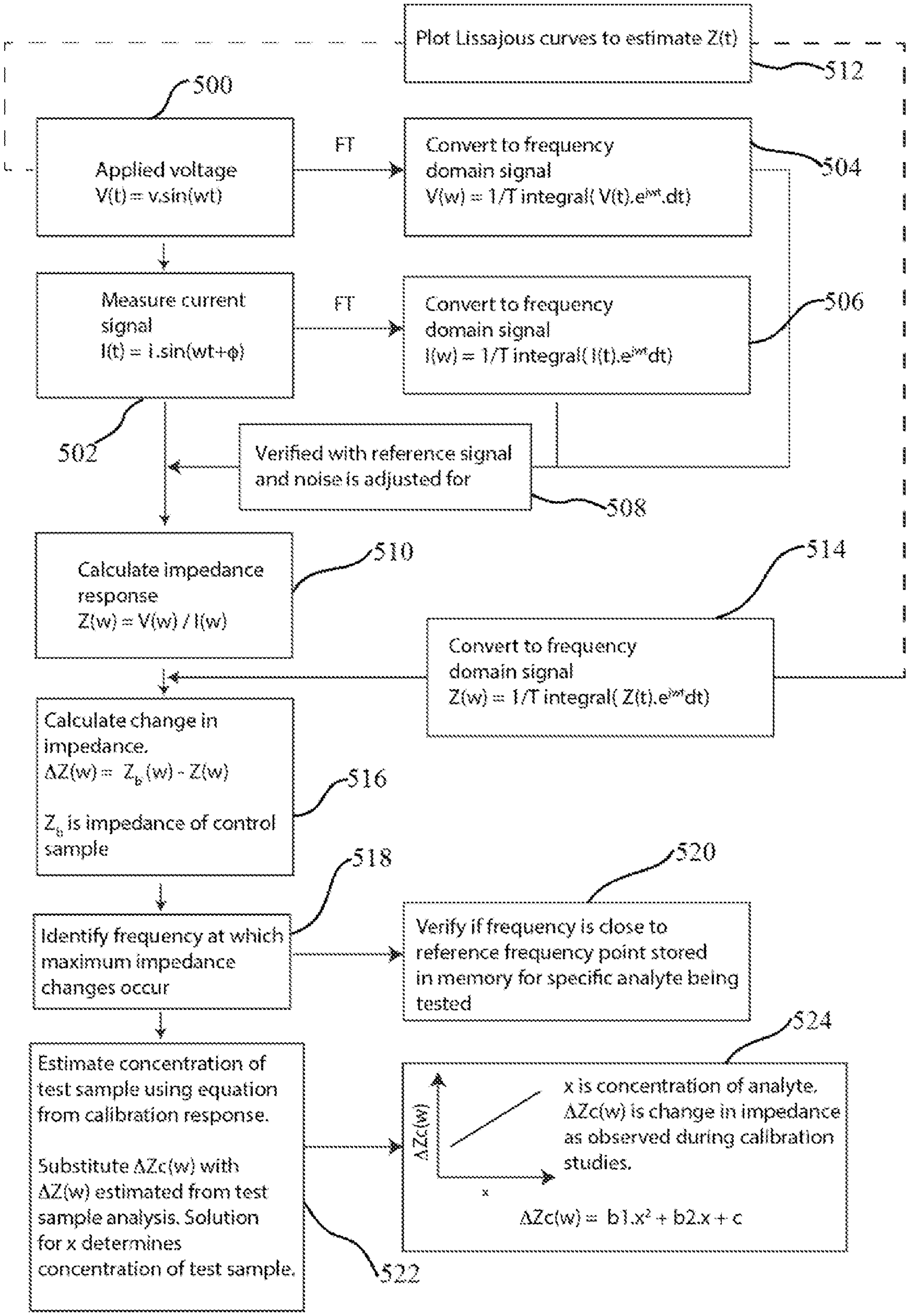
Plot Lissajous curves to estimate Z(t)
512
500
Applied voltage
V(t) = v.sin(wt)
FT
Convert to frequency domain signal
V(w) = 1/T integral( V(t).e^jwt.dt)
504
Measure current signal
I(t) = i.sin(wt+φ)
FT
Convert to frequency domain signal
I(w) = 1/T integral( I(t).e^jwt dt)
506
502
Verified with reference signal and noise is adjusted for
508
Calculate impedance response
Z(w) = V(w) / I(w)
510
514
Convert to frequency domain signal
Z(w) = 1/T integral( Z(t).e^jwt dt)
Calculate change in impedance.
ΔZ(w) = Z_b (w) - Z(w)
Z_b is impedance of control sample
516
518
520
Identify frequency at which maximum impedance changes occur
Verify if frequency is close to reference frequency point stored in memory for specific analyte being tested
524
Estimate concentration of test sample using equation from calibration response.
Substitute ΔZc(w) with ΔZ(w) estimated from test sample analysis. Solution for x determines concentration of test sample.
ΔZc(w)
x
x is concentration of analyte. ΔZc(w) is change in impedance as observed during calibration studies.
ΔZc(w) = b1.x² + b2.x + c
522

FIG. 11

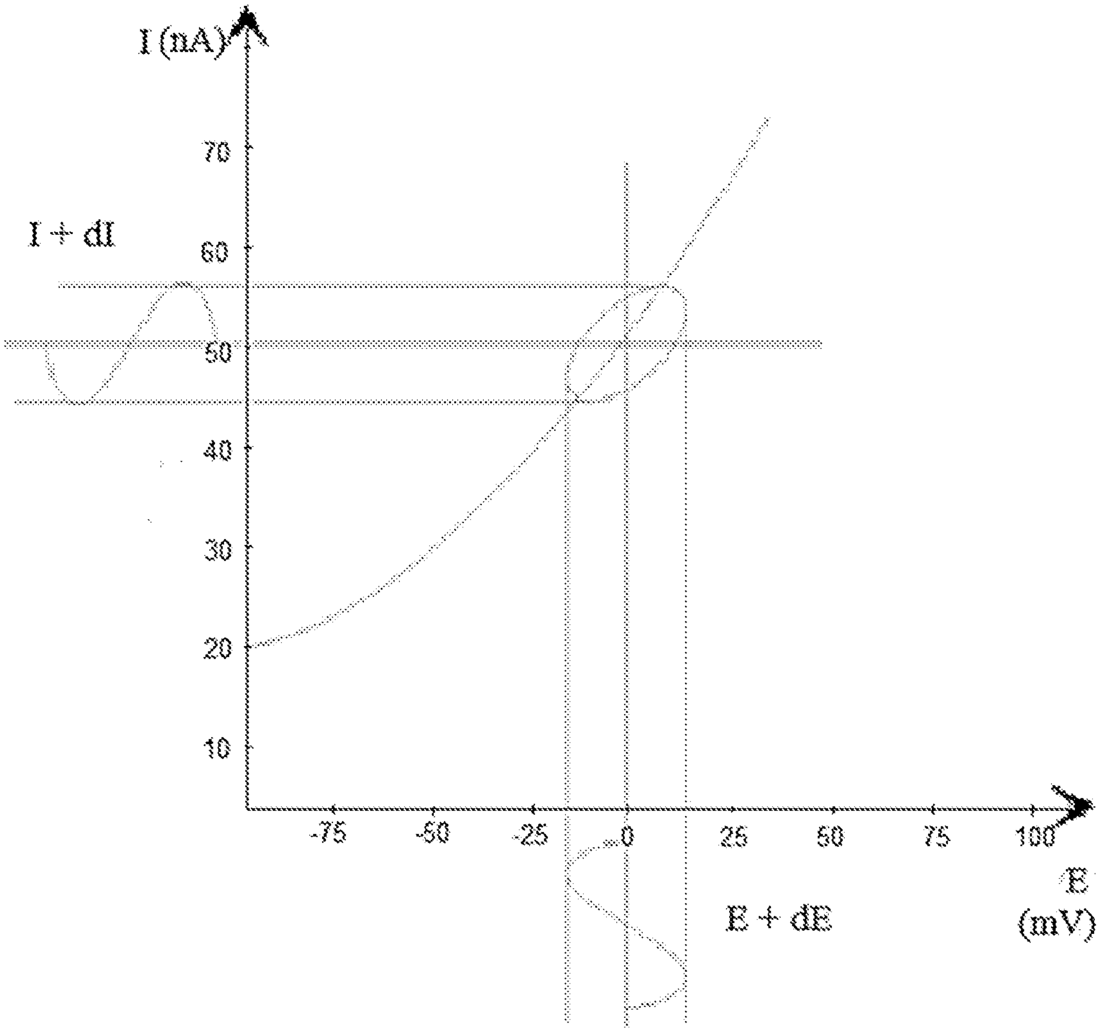


FIG. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BROEDERS, J. et al.** Miniturised eight-channel impedance spectroscopy unit as sensor platform for biosensor applications. *Physica Status Solidi A,* June 2011, vol. 208 (6), 1357-1363
- **BROEDERS, J. et al.** Mobile Application for Impedance-Based Biomimetic Sensor Readout. *IEEE Sensors Journal,* April 2013, vol. 13 (7), 2659-2665
- **SELVAM, A. P. et al.** Design of a high sensitive non-faradaic impedimetric sensor. *34th Annual International Conference of the IEEE EMBS,* September 2012, 3251-3254
- **ADAM C SIEGEL et al.** Foldable Printed Circuit Boards on Paper Substrates. *Adv. Funct. Mater.,* 2010, vol. 20, 28-35
- **ANNI MAATTANEN et al.** A low-cost paper-based inkjet-printed platform for electrochemical analyses. *Sensors and Actuators B,* 2013, vol. 177, 153-162
- **WIJITAR DUNGCHAI et al.** Electrochemical Detection for Paper-Based Microfluidics. *Anal. Chem.,* 2009, vol. 81, 5821-5826
- **REIGHARD ; BARENDT.** Conformal Coating Process Controls: The Manufacturing Engineer's Aid. *APEX. Long Beach, CA.,* March 2000
- **VESTERGAARD et al.** *Sensors,* 2007, vol. 7 (12), 3442-58